# EUROPEAN PATENT SPECIFICATION

(11) **EP 4 268 881 B1**
(45) Date of publication and mention of the grant of the patent: **29.07.2026**
(21) Application number: 21909921.5
(22) Date of filing: 20.10.2021
(51) Int. Cl.: A61M 25/10

(54) **BALLOON FOR BALLOON CATHETER**
BALLON FÜR BALLONKATHETER
BALLONNET POUR CATHÉTER À BALLONNET

(30) Priority: 24.12.2020 JP 2020215754
(43) Date of publication of application: 01.11.2023
(73) Proprietor: Kaneka Corporation, Osaka-shi, Osaka 530-8288 (JP)
(72) Inventor: KOJIMA, Masahiro, Settsu-shi, Osaka 566-0072 (JP); HAMABUCHI, Takahisa, Osaka-shi, Osaka 530-8288 (JP)
(74) Representative: Plasseraud IP
(86) International application number: PCT/JP2021/038820
(87) International publication number: WO 2022/137764

(56) References cited:
- WO-A1-2004/101057
- WO-A1-2020/012851
- WO-A1-2020/195170
- JP-A- 2008 529 740
- JP-A- 2016 052 452
- JP-A- 2016 520 369
- JP-A- 2018 102 353
- JP-B2- 6 166 658
- US-A- 6 013 055

## Description

### TECHNICAL FIELD

The present invention is set out in the appended claims and relates to a balloon for a balloon catheter and associated fabricating method.

### BACKGROUND ART

Diseases such as angina pectoris and myocardial infarction are caused by the formation of stenotic areas hardened by calcification and other factors in the inner walls of blood vessels. One of the treatments for these diseases is angioplasty, in which a balloon catheter is used to dilate the stenotic area. Angioplasty is a minimally invasive therapy that does not require an open chest procedure like bypass surgery and is widely used.

In angioplasty, it is sometimes difficult to dilate a stenosis that has hardened due to calcification and other factors with a standard balloon catheter. In some cases, while the method of dilating a stenosis by implanting an indwelling expansion device called a stent into the stenosis is also used, an ISR (In-Stent-Restenosis) lesion, for example, may occur after this treatment, in which the neointima of the vessel grows excessively and the vessel becomes stenotic again. The neointima in ISR lesions is soft and the surface is slippery, so a standard balloon catheter may cause the balloon to shift out of the lesion site during balloon dilation to damage the vessel.

Balloon catheters that can dilate a stenosis even in such calcified or ISR lesions include balloon catheters with a protrusion, blade, or scoring element on the balloon to bite into the stenosis. For example, Patent document 1 discloses a balloon catheter having a scoring element that is composed of a polymeric material stiffer than the polymeric material forming the balloon body, and that is flattened at one end and the other end of the balloon. Patent document 2 discloses a scoring balloon structure in which the height of the scoring element decreases along the tapered shape of the balloon, and Patent document 3 discloses a balloon catheter in which straight tubular part of the balloon has an outer protruding portion and the tapered part has an inner protruding portion. In the above Patent documents 1 to 3, the height of the scoring element decreases at ends of the balloon, or an inner protruding portion is provided instead of an outer protruding portion. On the other hand, there is a balloon catheter constructed such that the distal tapered part has a protruding portion higher than the protruding portion disposed at the straight tubular part of the balloon (Patent document 4).

### RELATED ART DOCUMENT

### PATENT DOCUMENT

Patent document 1: US 2016/0128718 A1
Patent document 2: JP 2014-506140 T
Patent document 3: WO2020/012851 disclosing all features according to the preamble of claim 1.
Patent document 4: WO2020/012850
Patent document 5: JP 6-166658 B2
Patent document 6: JP 2016-052452 A
Patent document 7: WO 2020/195170 A1.

### SUMMARY OF THE INVENTION

### PROBLEMS TO BE SOLVED BY THE INVENTION

A balloon catheter is inserted into a body cavity in a deflated and folded state and delivered to the treatment site. For this reason, the balloon catheters disclosed in the above Patent documents 1 to 3 attempt to improve the passage of the balloon by decreasing the height of the scoring element at the end part of the balloon to prevent the outer diameter from becoming large so that the balloon can be easily inserted into a body cavity. In the balloon catheter disclosed in the above Patent document 4, since only the distal cone region is introduced into the lesion and the balloon is inflated, the height of the protruding portion disposed at the distal tapered part is high so that the balloon can be inflated while cutting into the lesion with the element disposed at the distal cone region. However, none of these balloons were designed to incise a stenosis obliquely or to incise a wide area in a single movement while the balloon is moved forward or backward in the deflated state.

In view of the above circumstances, the objective of the present invention is to provide a balloon for a balloon catheter that can incise a stenosis obliquely or incise a stenosis widely in the deflated state during balloon delivery or when the balloon is delivered to a lesion.

### MEANS FOR SOLVING THE PROBLEMS

A balloon for a balloon catheter that can solve the above problem is according to claim 1 and has notably a balloon body having an outer surface and an inner surface; the balloon body has a straight tubular part, a distal tapered part located distal to the straight tubular part, and a proximal tapered part located proximal to the straight tubular part; the distal tapered part, the straight tubular part, and the proximal tapered part have a protrusion part that projects outwardly in a radial direction from the outer surface of the balloon body and extends in a longitudinal axis direction of the balloon body; the protrusion part has a tip part in a cross section in the radial direction of the balloon body; and the balloon satisfies at least one of the following (1) and (2):
(1) in a deflated state of the balloon for a balloon catheter, the tip part of the protrusion part of the distal tapered part is located on a first direction side and not on a second direction side, or located on the second direction side and not on the first direction side, in a circumferential direction of the balloon body with respect to a straight line L_{d} connecting the tip part at a proximal end of the distal tapered part and the tip part at a distal end of the distal tapered part; and
(2) in a deflated state of the balloon for a balloon catheter, the tip part of the protrusion part of the proximal tapered part is located on a first direction side and not on a second direction side, or located on the second direction side and not on the first direction side, in a circumferential direction of the balloon body with respect to a straight line Lₚ connecting the tip part at a distal end of the proximal tapered part and the tip part at a proximal end of the proximal tapered part. According to the present invention, the latter balloon for a balloon catheter is further characterized in that the tip part of the protrusion part of at least one of the distal tapered part and the proximal tapered part is arranged curved on the first direction side or the second direction side in the circumferential direction of the balloon body independently with respect to the straight line Ld and the straight line Lp , respectively in the deflated state of the balloon.

Preferably, the balloon for a balloon catheter is folded in the deflated state of the balloon for a balloon catheter.

Preferably, when ends of the distal tapered part and the proximal tapered part on the straight tubular part side are termed as a position of 0% and the other ends of the distal tapered part and the proximal tapered part are termed as a position of 100% in the longitudinal axis direction of the balloon body in the deflated state of the balloon for a balloon catheter, the balloon satisfies at least one of the following (1) and (2):
(1) the tip part of the protrusion part in the entire extent of a section from a position of 20% to a position of 70% of the distal tapered part is located on the first direction side or on the second direction side with respect to the straight line L_{d}, and the tip part of the protrusion part in a section from a position of 90% to the position of 100% of the distal tapered part is not located on either the first direction side or the second direction side with respect to the straight line L_{d}; and
(2) the tip part of the protrusion part in the entire extent of a section from a position of 20% to a position of 70% of the proximal tapered part is located on the first direction side or on the second direction side with respect to the straight line Lₚ, and the tip part of the protrusion part in a section from a position of 90% to the position of 100% of the proximal tapered part is not located on either the first direction side or the second direction side with respect to the straight line Lₚ.

In this case, the balloon preferably satisfies at least one of the following (1) and (2):
(1) a distance from the straight line L_{d} to the tip part of the protrusion part at a position of 40% of the distal tapered part is 1.2 times or longer than a distance from the straight line L_{d} to the tip part of the protrusion part at a position of 60% of the distal tapered part; and
(2) a distance from the straight line Lₚ to the tip part of the protrusion part at a position of 40% of the proximal tapered part is 1.2 times or longer than a distance from the straight line Lₚ to the tip part of the protrusion part at a position of 60% of the proximal tapered part.

Preferably, the balloon satisfies at least one of the following (1) and (2) in the deflated state of the balloon for a balloon catheter:
(1) the tip part of the protrusion part in the entire extent of a section from a position of 20% to a position of 70% of the distal tapered part is located inside or at the same position in the radial direction of the balloon body with respect to an imaginary curved surface obtained by rotating the straight line L_{d} around a central axis of the balloon body; and
(2) the tip part of the protrusion part in the entire extent of a section from a position of 20% to a position of 70% of the proximal tapered part is located inside or at the same position in the radial direction of the balloon body with respect to an imaginary curved surface obtained by rotating the straight line Lₚ around a central axis of the balloon body.

Preferably, the tip part of the protrusion part of the distal tapered part, the tip part of the protrusion part of the straight tubular part, and the tip part of the protrusion part of the proximal tapered part are located at the same position in the circumferential direction of the balloon body in an inflated state of the balloon for a balloon catheter.

Preferably, the balloon body has a wing forming portion that forms a wing in the deflated state, and the protrusion part is located outside the wing forming portion.

Preferably, the protrusion part of the distal tapered part, the protrusion part of the straight tubular part, and the protrusion part of the proximal tapered part extend continuously in the longitudinal axis direction of the balloon body.

Preferably, the protrusion part is composed of the same material as the balloon body.

The present invention also provides a method ,as defined in claim 10, for producing the above-described balloon for a balloon catheter. A method in accordance with one embodiment of the present invention comprises: preparing a first tubular member, a second tubular member, and a third tubular member, each of which has an inner space extending in a longitudinal axis direction; preparing a balloon for a balloon catheter having a balloon body having an outer surface and an inner surface, the balloon body having a straight tubular part, a distal tapered part located distal to the straight tubular part, and a proximal tapered part located proximal to the straight tubular part, and the distal tapered part, the straight tubular part, and the proximal tapered part having a protrusion part that projects outwardly in a radial direction from the outer surface of the balloon body and extends in a longitudinal axis direction of the balloon body; and placing the distal tapered part in the first tubular member, placing the proximal tapered part in the second tubular member, and placing the straight tubular part in the third tubular member in the deflated state of the balloon for a balloon catheter, wherein the method satisfies at least one of the following (1) and (2):
(1) at least a part of the protrusion part of the distal tapered part is in contact with an inner surface of the first tubular member in the placing process; and
(2) at least a part of the protrusion part of the proximal tapered part is in contact with an inner surface of the second tubular member in the placing process.

### EFFECTS OF THE INVENTION

The above balloon for a balloon catheter can incise a stenosis obliquely or incise a wide region while the balloon is moved forward or backward in the deflated state during balloon delivery or when the balloon is delivered to a lesion, because at least one of the tip part of the protrusion part of the distal tapered part and the proximal tapered part is located on the first direction side and not on the second direction side, or located on the second direction side and not on the first direction side, in the circumferential direction of the balloon.

### BRIEF DESCRIPTION OF THE DRAWINGS

FIG. 1 is a side view of a balloon catheter in accordance with one embodiment of the present invention.
FIG. 2 is a cross-sectional view of the balloon of the balloon catheter shown in FIG. 1 in the inflated state in the longitudinal direction.
FIG. 3 is a III-III cross-sectional view of FIG. 1.
FIG. 4 is a cross-sectional view of the straight tubular part of the balloon shown in FIG. 2 in the deflated state in the radial direction.
FIG. 5 is a cross-sectional view of the tapered part of the balloon shown in FIG. 2 in the deflated state in the radial direction.
FIG. 6 is a plan view of the balloon shown in FIG. 2 in the deflated state seeing from the protrusion part side.
FIG. 7 is a plan view of a balloon in accordance with another embodiment of the present invention in the deflated state seeing from the protrusion part side.
FIG. 8 is a plan view of a balloon in accordance with still another embodiment of the present invention in the deflated state seeing from the protrusion part side.
FIG. 9 is a plan view of a balloon in accordance with still another embodiment of the present invention in the deflated state seeing from the protrusion part side.
FIG. 10 is a plan view of a balloon in accordance with still another embodiment of the present invention in the deflated state seeing from the protrusion part side.
FIG. 11 is a plan view of a balloon in accordance with still another embodiment of the present invention in the deflated state seeing from the protrusion part side.
FIG. 12 is a plan view of a balloon in accordance with still another embodiment of the present invention in the deflated state seeing from the protrusion part side.
FIG. 13 is a plan view of a balloon in accordance with still another embodiment of the present invention in the deflated state seeing from the protrusion part side.
FIG. 14 is a side view of the balloon shown in FIG. 2 in the folded state.
FIG. 15 is a XV-XV cross-sectional view of FIG. 14.
FIG. 16 is a XVI-XVI cross-sectional view of FIG. 14.
FIG. 17 is a XVII-XVII cross-sectional view of FIG. 14.
FIG. 18 is a plan view of a balloon in accordance with another embodiment of the present invention in the deflated state seeing from the protrusion part side.
FIG. 19 is a plan view of a balloon in accordance with still another embodiment of the present invention in the deflated state seeing from the protrusion part side.
FIG. 20 is a partial side view of the balloon shown in FIG. 2 in the deflated state.
FIG. 21 is a plan view of the balloon shown in FIG. 2 seeing from the protrusion part side.
FIG. 22 is a perspective view of a parison before expansion in accordance with one embodiment of the present invention.
FIG. 23 is a cross-sectional view perpendicular to the longitudinal axis direction of a first tubular member and a second tubular member of a producing method in accordance with one embodiment of the present invention.
FIG. 24 is a radial cross-sectional view in a process of placing the distal tapered part in the first tubular member or a process of placing the proximal tapered part in the second tubular member in the producing method in accordance with one embodiment of the present invention.
Fig. 25 is a radial cross-sectional view of the first tubular member or the second tubular member shown in FIG. 24 at different position in the longitudinal axis direction.
FIG. 26 is a radial cross-sectional view of the first tubular member or the second tubular member shown in FIG. 24 at still different position in the longitudinal axis direction.
FIG. 27 is a radial cross-sectional view of a third tubular member of the producing method in accordance with one embodiment of the present invention.

### MODE FOR CARRYING OUT THE INVENTION

Hereinafter, the present invention as defined in the appended claims will be described in detail based on the following embodiments, but the present invention is not limited by the following embodiments . Note that, in each drawing, hatching, reference signs for components, and the like may be omitted for convenience of description, and in such a case, the specification and other drawings are to be referred to. Furthermore, since the dimensions of the various components in the drawings are provided for the purpose of facilitating the understanding of the feature of the present invention, the dimensions may differ from the actual dimensions in some cases.

A balloon for a balloon catheter in accordance with one embodiment of the present invention has a balloon body having an outer surface and an inner surface; the balloon body has a straight tubular part, distal tapered part located distal to the straight tubular part, and a proximal tapered part located proximal to the straight tubular part; the distal tapered part, the straight tubular part, and the proximal tapered part have a protrusion part that projects outwardly in a radial direction from the outer surface of the balloon body and extends in a longitudinal axis direction of the balloon body; the protrusion part has a tip part in a cross section in the radial direction of the balloon body; and the balloon satisfies at least one of the following (1) and (2):
(1) in a deflated state of the balloon for a balloon catheter, the tip part of the protrusion part of the distal tapered part is located on a first direction side and not on a second direction side, or located on the second direction side and not on the first direction side, in a circumferential direction of the balloon body with respect to a straight line L_{d} connecting the tip part at a proximal end of the distal tapered part and the tip part at a distal end of the distal tapered part; and
(2) in a deflated state of the balloon for a balloon catheter, the tip part of the protrusion part of the proximal tapered part is located on a first direction side and not on a second direction side, or located on the second direction side and not on the first direction side, in a circumferential direction of the balloon body with respect to a straight line Lₚ connecting the tip part at a distal end of the proximal tapered part and the tip part at a proximal end of the proximal tapered part.

Because the tip part of the protrusion part of at least one of the distal tapered part and the proximal tapered part is located on the first direction side or the second direction side in the circumferential direction of the balloon body in the deflated state of the balloon, the balloon can incise a stenosis obliquely or incise a wide area in a single movement while the balloon is moved forward or backward in the deflated state. In the present specification, the balloon for a balloon catheter may be referred to simply as the "balloon".

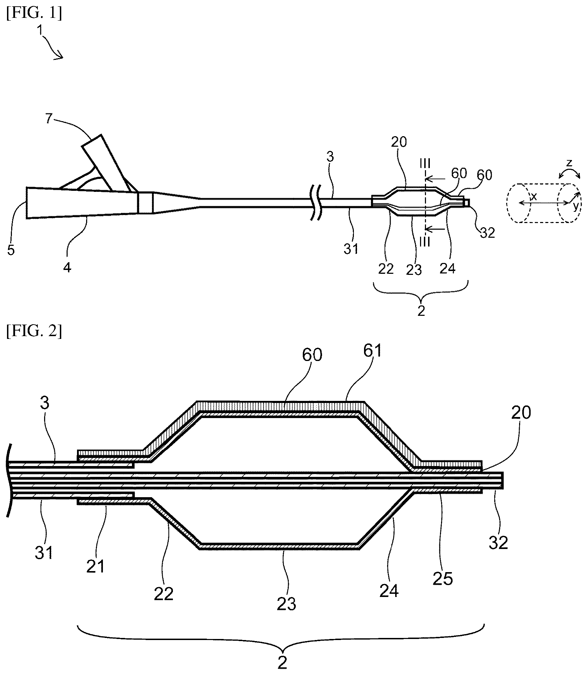

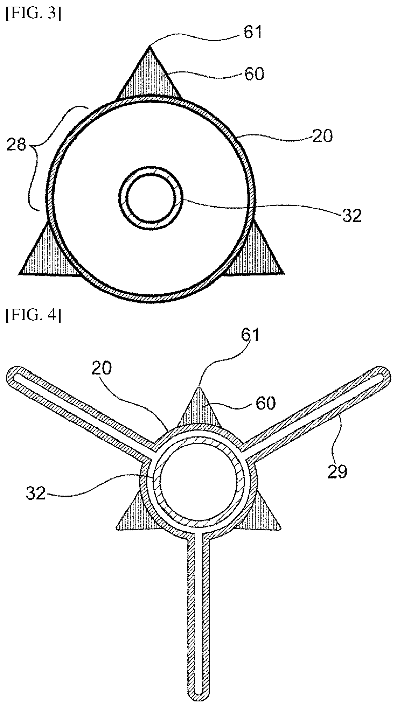

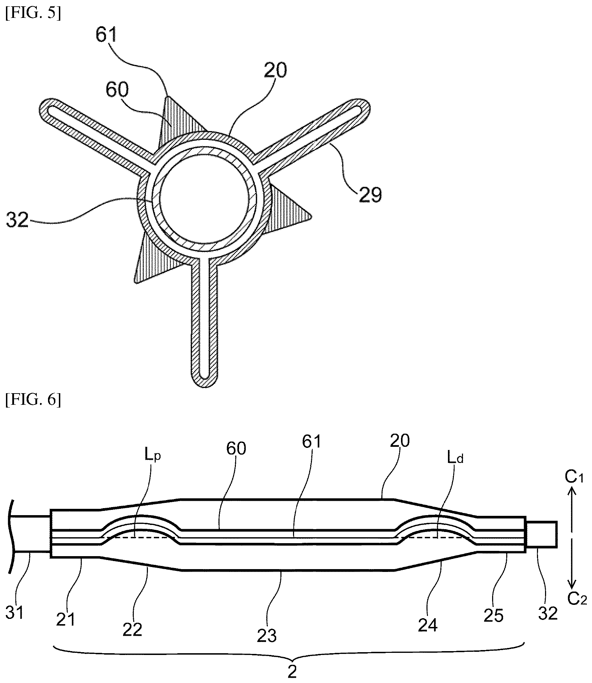

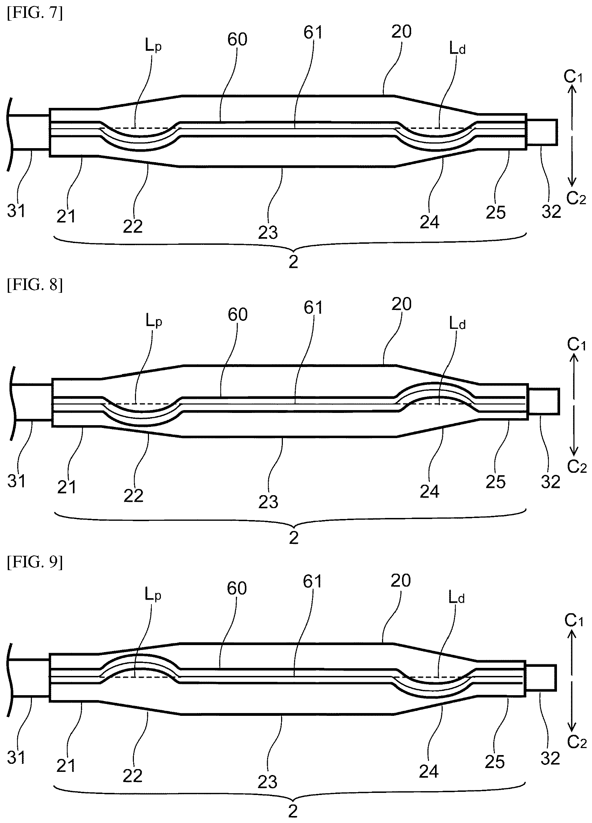

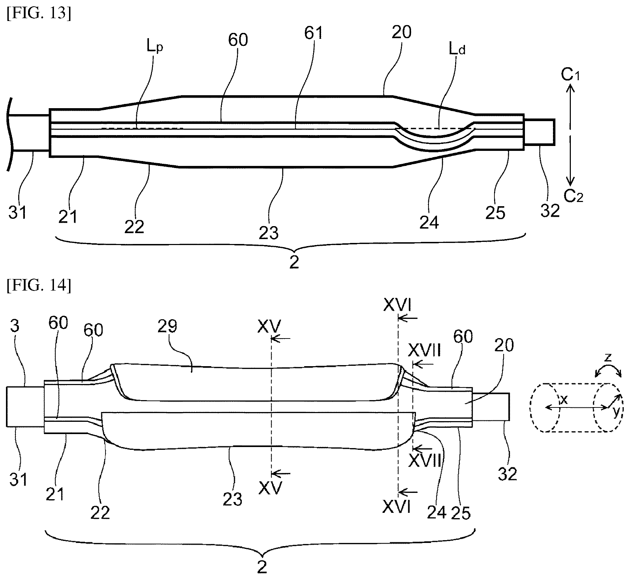

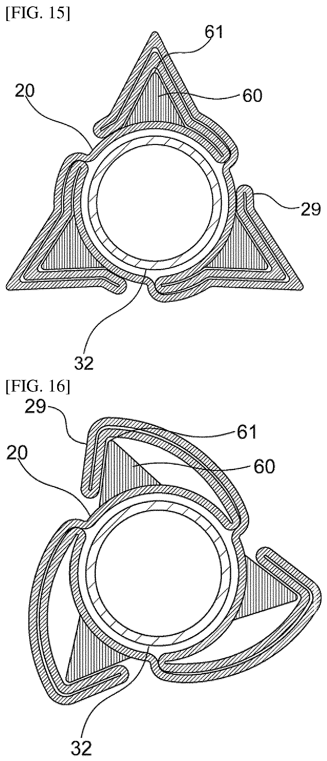

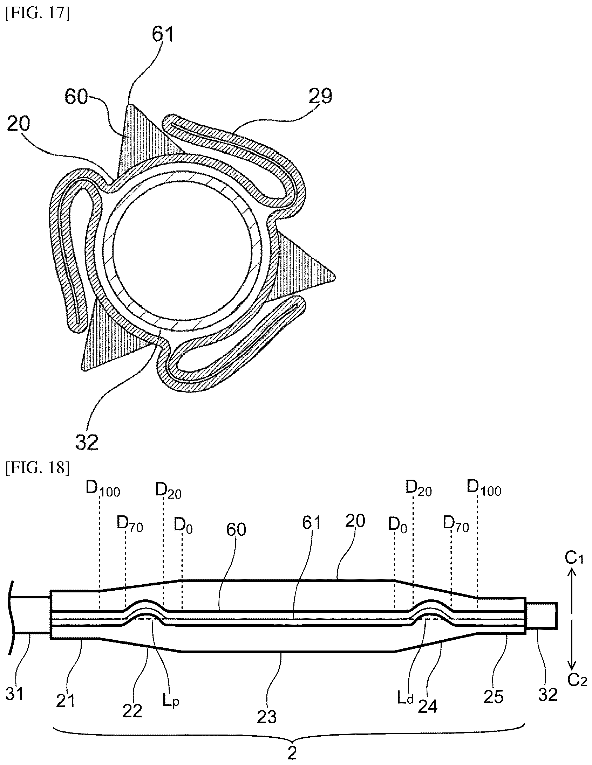

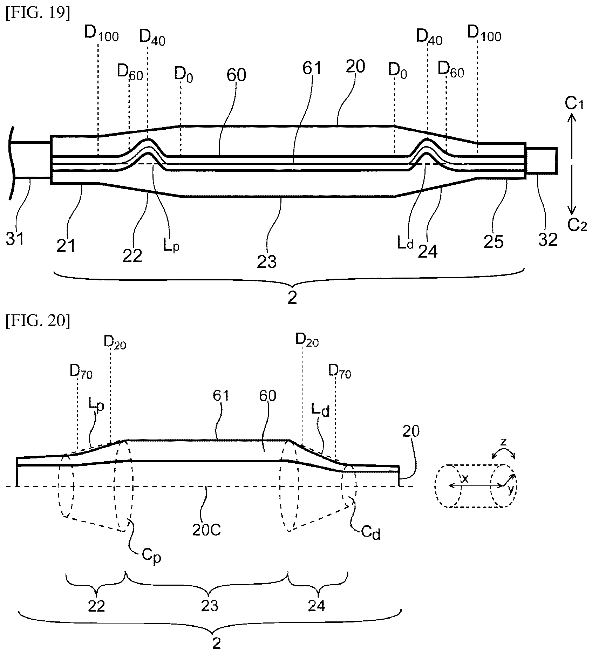

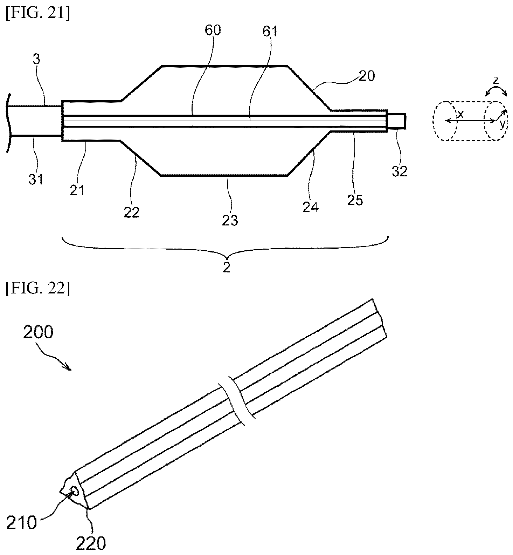

Referring to FIG. 1 to FIG. 21, the balloon for a balloon catheter will be explained. FIG. 1 is a side view of a balloon catheter in accordance with one embodiment of the present invention, FIG. 2 is a cross-sectional view of the balloon of the balloon catheter shown in FIG. 1 in the inflated state in the longitudinal direction, and FIG. 3 is a III-III cross-sectional view of FIG. 1. FIG. 4 is a cross-sectional view of the straight tubular part of the balloon shown in FIG. 2 in the deflated state in the radial direction, and FIG. 5 is a cross-sectional view of the tapered part of the balloon shown in FIG. 2 in the deflated state in the radial direction. FIG. 6 is a plan view of the balloon shown in FIG. 2 in the deflated state seeing from the protrusion part side, and FIGs 7 to 13 are plan views of a balloon in accordance with different embodiments of the present invention in the deflated state seeing from the protrusion part side. FIG. 14 is a side view of the balloon shown in FIG. 2 in the folded state, and FIG. 15, FIG. 16, and FIG. 17 is a XV-XV cross-sectional view, XVI-XVI cross-sectional view, and XVII-XVII cross-sectional view of FIG. 14, respectively. FIGs 18 and 19 are plan views of a balloon in accordance with different embodiments of the present invention in the deflated state seeing from the protrusion part side. FIG. 20 is a partial side view of the balloon shown in FIG. 2 in the deflated state, that is, a view of a part in which a protrusion part is formed along the balloon body seeing from directly across. FIG. 21 is a plan view of the balloon shown in FIG. 2 seeing from the protrusion part side.

In the present invention, a proximal side refers to the direction towards a user's or operator's hand in the extending direction of a balloon catheter 1 or the longitudinal axis direction x of a shaft 3, and a distal side refers to the opposite side of the proximal side, that is, the direction towards the person to be treated. Members other than long-shaped members, such as the shaft 3, also has the same longitudinal axis direction x as the shaft 3. A radial direction y is the direction perpendicular to the longitudinal axis direction x, connecting the center of a balloon body 20 and a point on the circumscribed circle of the balloon body 20 in a cross section perpendicular to the longitudinal axis direction x. A circumferential direction z is the direction along the circumference of the circumscribed circle of the balloon body 20 in the inflated state in a cross section perpendicular to the longitudinal axis direction x.

As shown in FIGs 1 and 2, a balloon catheter 1 has a shaft 3 and a balloon 2 disposed outside the shaft 3. The balloon catheter 1 has the distal side and the proximal side, and the balloon 2 is disposed on the distal side of the shaft 3. The balloon catheter 1 is configured such that fluid is introduced in the balloon 2 through the shaft 3, and the inflation and deflation of the balloon 2 can be controlled using an indeflator (pressurizer for balloons). The fluid may be a pressurized fluid pressurized by a pump or the like.

The shaft 3 preferably has a flow path for the fluid inside, and further has a guidewire insertion path. Configuration in which the shaft 3 has an internal fluid path and guidewire insertion path include, for example, a configuration in which the shaft 3 has an outer tube 31 and an inner tube 32, and the inner tube 32 serves as the guidewire insertion path and the space between the inner tube 32 and the outer tube 31 serves as the fluid flow path. In the case where the shaft 3 has the outer tube 31 and the inner tube 32, preferably, the inner tube 32 extends through the distal end of the outer tube 31 and penetrates the balloon 2 to the distal side, the distal side of the balloon 2 is fixed to the inner tube 32, and the proximal side of the balloon 2 is fixed to the outer tube 31.

As shown in FIGs 1 to 13, the balloon 2 for a balloon catheter 1 has the balloon body 20 having an outer surface and inner surface; the balloon body 20 has a straight tubular part 23, a distal tapered part 24 located distal to the straight tubular part 23, and a proximal tapered part 22 located proximal to the straight tubular part 23; the distal tapered part 24, the straight tubular part 23, and the proximal tapered part 22 have a protrusion part 60 that projects outwardly in the radial direction from the outer surface of the balloon body 20 and extend in the longitudinal axis direction x; the protrusion part 60 has a tip part 61 in a cross section in the radial direction y of the balloon body 20; and the balloon 2 satisfies at least one of the following (1) and (2):
(1) in a deflated state of the balloon 2 for the balloon catheter 1, the tip part 61 of the protrusion part 60 of the distal tapered part 24 is located on a first direction C₁ side and not on a second direction C₂ side, or located on the second direction C₂ side and not on the first direction C₁ side, in a circumferential direction z of the balloon body 20 with respect to a straight line L_{d} connecting the tip part 61 at a proximal end of the distal tapered part 24 and the tip part 61 at a distal end of the distal tapered part 24; and
(2) in a deflated state of the balloon 2 for a balloon catheter 1, the tip part 61 of the protrusion part 60 of the proximal tapered part 22 is located on a first direction C₁ side and not on a second direction C₂ side, or located on the second direction C₂ side and not on the first direction C₁ side, in a circumferential direction z of the balloon body 20 with respect to a straight line Lₚ connecting the tip part 61 at a distal end of the proximal tapered part 22 and the tip part 61 at a proximal end of the proximal tapered part 22.

In the deflated state of the balloon 2, the tip part 61 of the protrusion part 60 in at least one of the distal tapered part 24 and the proximal tapered part 22 is located on the first direction C₁ side or the second direction C₂ side in the circumferential direction z of the balloon body 20, and therefore, it is possible to incise the stenosis obliquely or incise a wide area with a single movement while moving the balloon 2 forward or backward in the deflated state during delivery of the balloon 2 or when the balloon 2 is delivered to the lesion.

As shown in FIG. 2, the balloon 2 may have a distal sleeve part 25 and a proximal sleeve part 21, which are non-inflatable, on the side distal to the distal tapered part 24 and proximal to the proximal tapered part 22, respectively. The distal sleeve part 25 and the proximal sleeve part 21 can be at least partially fixed to the shaft 3. When the shaft 3 has the outer tube 31 and the inner tube 32, at least a part of the proximal sleeve part 21 can be fixed to the outer tube 31 and at least a part of the distal sleeve part 25 can be fixed to the inner tube 32.

The distal tapered part 24 and the proximal tapered part 22 are preferably formed so that the diameter decreases as it is away from the straight tubular part 23. When the balloon 2 is inflated at a stenosis, the balloon body 20 having the straight tubular part 23 having the largest diameter in the inflated state allows the straight tubular part 23 to make sufficient contact with the stenosis to facilitate dilation or incise of the stenosis. In addition, while a wing 29 is formed when the balloon 2 is deflated as described below, the balloon body 20 having the distal tapered part 24 and the proximal tapered part 22 whose outer diameter decrease as they move away from the straight tubular part 23 allows the protrusion part 60 to be exposed from the wing 29 of the balloon 2 at the distal tapered part 24 and the proximal tapered part 22 when the balloon 2 is deflated and the wing 29 is wrapped around the shaft 3, and the exposed protrusion part 60 can incise the stenosis even when the balloon 2 is deflated.

As shown in FIG. 2 and FIG. 3, the protrusion part 60 of the balloon 2 is a part that projects outwardly in the radial direction y from the outer surface of the balloon body 20 in the inflated state of the balloon 2. The maximum length of the protrusion part 60 projecting outwardly in the radial direction y from the outer surface of the balloon body 20 in a cross section in the radial direction y is preferably 1.2 times or more the film thickness of the balloon body 20, more preferably 1.5 times or more, even more preferably 2 times or more, and is acceptable to be 100 times or less, 50 times or less, 30 times or less, or 10 times or less. This makes it easier to make an incision of appropriate depth in the stenosis with the protrusion part 60 and facilitate incision. Such a protrusion part 60 also makes it possible to improve the strength of the balloon 2 and prevent overinflation of the balloon 2 when pressurized.

The number of the protrusion part 60 in the circumferential direction z of the balloon 2 may be one, or more than one as shown in FIG. 3. When the balloon 2 has a plurality of the protrusion part 60 in the circumferential direction z, each protrusion part 60 is preferably spaced apart from each other in the circumferential direction z, and more preferably equally spaced in the circumferential direction z. A plurality of the protrusion part 60, each of which is spaced apart, preferably equally spaced, allows the balloon 2 to be easily fixed and facilitates incision of the stenosis.

As shown in FIG. 3, the protrusion part 60 has the tip part 61 in a cross section in the radial direction y of the balloon body 20. The tip part 61 makes it easier to make an incision into the stenosis, thereby preventing dissection of the vascular intima to incise the stenosis. The tip part 61 is a part of the protrusion part 60, and the tip part 61 projects most outwardly in the radial direction y from the outer surface of the balloon body 20, and may have a shape with an acute angle as shown in FIG. 3, or may have a shape with an obtuse angle, a shape comprising a curve, or a flat shape. From the point of view of ease of incision, it is preferable to have a shape with an acute angle. The cross-sectional shape of the protrusion part 60 in the radial direction y may be any shape, and may be approximate triangle as shown in FIG. 3, or polygon, fan shape, wedge shape, convex shape, spindle shape, or the like.

As shown in FIG. 4 and FIG. 5, the deflated state of the balloon 2 is a state after fluid has been discharged from the inside of the balloon 2 or before fluid is introduced into the inside of the balloon 2. In the deflated state of the balloon 2, the wing 29 and a portion where the inner surface of the balloon body 20 is in close proximity to the shaft 3 are formed. In other words, as shown in FIG. 3, the balloon 2 in the inflated state has a wing forming portion 28 that is formed into the wing 29 in the deflated state. In the embodiments shown in FIG. 4 and FIG. 5, the shaft 3 has the outer tube 31 and the inner tube 32, and the balloon 2 has a portion where the inner surface of the balloon body 20 is in close proximity to the inner tube 32 in the deflated state. As can be understood by comparing FIG. 4, which shows a cross-sectional view of the straight tubular part 23 in the radial direction y in the deflated state, and FIG. 5, which shows a cross-sectional view of the tapered part (the distal tapered part 24 or the proximal tapered part 22) in the radial direction y in the deflated state, in a cross section in the radial direction y, the length of the wing 29 of the straight tubular part 23 in the radial direction y is longer than the length of the wing 29 of the tapered part in the radial direction y, because the straight tubular part 23 is a part having the largest diameter and the tapered part is a part having a reduced diameter in the inflated state. When the distal tapered part 24 and the proximal tapered part 22 are gradually reduced in diameter toward the distal and proximal sides, respectively, the length of the wing 29 in the radial direction y in a cross section in the radial direction y is also gradually reduced toward the distal and proximal sides, respectively, and the wing 29 may not by formed at a distal side part of the distal tapered part 24 and a proximal side part of the proximal tapered part 22. Preferably, the wing 29 is not formed at a distal end part of the distal tapered part 24 and a proximal end part of the proximal tapered part 22. When the wing 29 is not formed at the distal end part of the distal tapered part 24 and the proximal end part of the proximal tapered part 22, the protrusion part 60 can contact the body cavity wall without being impeded by the wing 29 at those parts, allowing the incision of the stenosis.

As shown in FIGs 6 to 13, in the deflated state of the balloon 2, the tip part 61 of the protrusion part 60 in at least one of the distal tapered part 24 and the proximal tapered part 22 is, independently, located on the first direction C₁ side and not on the second direction C₂ side in the circumferential direction z of the balloon body 20 with respect to the straight line L_{d} and the straight line Lₚ, respectively, or located on the second direction C₂ side and not on the first direction C₁ side. In other words, the tip part 61 of the protrusion part 60 in at least one of the distal tapered part 24 and the proximal tapered part 22 is not disposed on either side across the straight line L_{d} and the straight line Lₚ. While FIGs 6 to 13 show embodiments in which the tip part 61 of the protrusion part 60 in the entire extent from the distal end to the proximal end of at least one of the distal tapered part 24 and the proximal tapered part 22 is placed on the first direction C₁ side or the second direction C₂ side, the tip part 61 of the protrusion part 60 of at least one of the distal tapered part 24 and the proximal tapered part 22 only needs to have a part located on the first direction C₁ side or the second direction C₂ side, and the rest may be located on the straight line L_{d} or the straight line Lₚ.

As shown in FIG. 6 and FIG. 7, the tip part 61 of the protrusion part 60 of both the distal tapered part 24 and the proximal tapered part 22 may be located on the first direction C₁ side or the second direction C₂ side with respect to the straight line L_{d} and the straight line Lₚ. These configurations allow the tip part 61 of the protrusion part 60 at the distal tapered part 24 and the tip part 61 of the protrusion part 60 at the proximal tapered part 22 to be located on the same side of the circumferential direction z, allowing the tip part 61 to be located on the same side of the circumferential direction z when the balloon 2 is advanced or retracted in the body cavity. Thus, the same side of the body cavity wall in the circumferential direction z can be incised obliquely or a wide region can be incised with a single movement using the tip part 61 of the protrusion part 60 of both the distal tapered part 24 and the proximal tapered part 22.

As shown in FIG. 8 and FIG. 9, the tip part 61 of the protrusion part 60 of the distal tapered part 24 may be located on the first direction C₁ side or the second direction C₂ side with respect to the straight line L_{d}, and the tip part 61 of the protrusion part 60 of the proximal tapered part 22 may be located, contrary to the distal tapered part 24, on the second direction C₂ side or the first direction C₁ side with respect to the straight line Lₚ. These configurations allow the tip part 61 of the protrusion part 60 at the distal tapered part 24 and the tip part 61 of the protrusion part 60 at the proximal tapered part 22 to be located on different sides of the circumferential direction z, allowing the tip part 61 to be located on different sides of the circumferential direction z when the balloon 2 is advanced or retracted in the body cavity. Thus, different part of the body cavity wall can be incised obliquely depending on whether the balloon 2 moves forward or backward, or a wider region can be incised with a single movement.

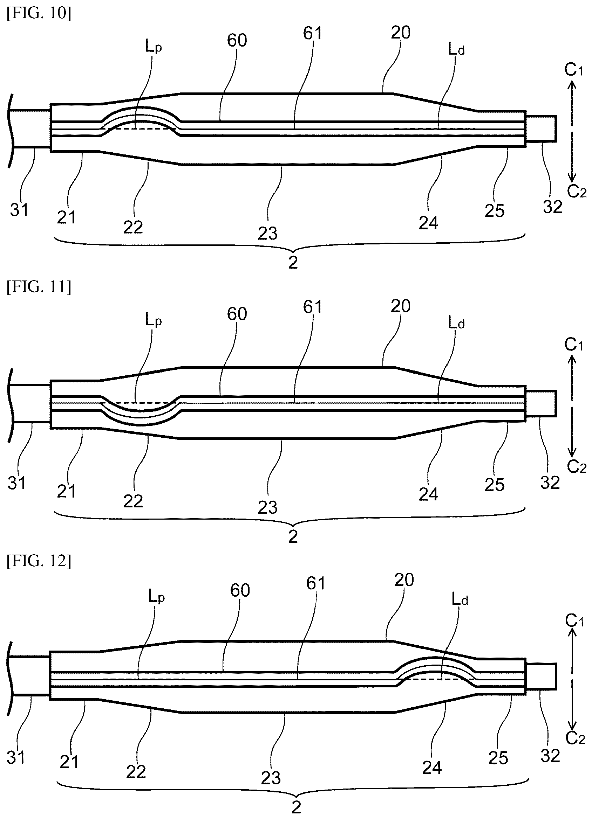

As shown in FIG. 10 and FIG. 11, the tip part 61 of the protrusion part 60 of the proximal tapered part 22 may be located on the first direction C₁ side or the second direction C₂ side with respect to the straight line Lₚ, and the tip part 61 of the protrusion part 60 of the distal tapered part 24 may not be located on either the first direction C₁ side or the second direction C₂ side with respect to the straight line L_{d}. These configurations allow only the tip part 61 of the protrusion part 60 at the proximal tapered part 22 to be located on the first direction C₁ side or the second direction C₂ side in the circumferential direction z, and the tip part 61 of the protrusion part 60 of the distal tapered part 24 not to be curved in the circumferential direction z. Thus, the tip part 61 of the distal tapered part 24 can incise straightly, and the tip part 61 of the proximal tapered part 22 can incise obliquely of incise a wide region in a single movement while the balloon 2 is, for example, moved backward.

As shown in FIG. 12 and FIG. 13, the tip part 61 of the protrusion part 60 of the distal tapered part 24 may be located on the first direction C₁ side or the second direction C₂ side with respect to the straight line L_{d}, and the tip part 61 of the protrusion part 60 of the proximal tapered part 22 may not be located on either the first direction C₁ side or the second direction C₂ side with respect to the straight line Lₚ. These configurations allow only the tip part 61 of the protrusion part 60 at the distal tapered part 24 to be located on the first direction C₁ side or the second direction C₂ side in the circumferential direction z, and the tip part 61 of the protrusion part 60 of the proximal tapered part 22 not to be curved in the circumferential direction z. Thus, the tip part 61 of the proximal tapered part 22 can incise straightly, and the tip part 61 of the distal tapered part 24 can incise obliquely or incise a wide region in a single movement while the balloon 2 is, for example, moved forward.

As described above, the balloon 2 can be made most suitable for treating depending on a lesion to be treated by selecting various configurations as shown, for example, in FIGs 6 to 13. While FIGs 6 to 13 show the embodiments in which the straight line L_{d} and the straight line Lₚ extend in the longitudinal axis direction x, the straight line L_{d} and the straight line Lₚ may extend at an angle in the circumferential direction z with respect to the longitudinal axis direction x.

In addition, while FIGs 6 to 13 show the embodiments in which the distal sleeve part 25 and the proximal sleeve part 21 also have the protrusion part 60, the distal sleeve part 25 and the proximal sleeve part 21 may not have the protrusion part 60, and may have an inner protrusion part that projects inwardly in the radial direction y from the inner surface of the balloon body 20. The distal sleeve part 25 and the proximal sleeve part 21 without the protrusion part 60 can make it easier for the balloon 2 to be inserted into the body cavity and moved forward or backward.

Regarding the length of the protrusion part 60 in the radial direction y in a cross section in the radial direction y, the length of the protrusion part 60 at the distal tapered part 24 and the proximal tapered part 22 is preferably shorter than the length of the protrusion part 60 at the straight tubular part 23. In addition, preferably, the tip part 61 of the protrusion part 60 at the distal tapered part 24 and the proximal tapered part 22 is not placed outward with respect to the straight line L_{d} and Lₚ in the inflated state. This can reduce the risk that the tip part 61 of the protrusion part 60 at the tapered parts other than the straight tubular part 23 acting on the lesion contacts a part that is not the target of treatment, such as a normal blood vessel, in the inflated state of the balloon 2.

As shown in FIGs 14 to 17, the balloon 2 is preferably folded in the deflated state of the balloon 2. In the folded state of the balloon 2, the wing 29 formed by the deflation of the balloon 2 shown in FIG. 4 and FIG. 5 is wrapped around the shaft 3 in a circumferential manner. Since the straight tubular part 23 having the maximum diameter has the wing 29 the length of which in the radial direction y is long, the wrapping amount of the wing 29 is larger as shown in FIG. 15. On the other hand, in the distal tapered part 24 and the proximal tapered part 22 that have reduced diameter, the wing 29 has a shorter length in the radial direction y due to the reduced diameter, and in one embodiment of the present invention, the length is shorter toward the more distal and more proximal side. In one embodiment of the present invention, the wing 29 having shorter length at a part of the distal tapered part 24 and the proximal tapered part 22 close to the straight tubular part 23 than at the straight tubular part 23 is wrapped around the shaft 3 (inner tube 32) in a circumferential manner as shown in FIG. 16, and at a more distal or more proximal side, the wing 29 having still shorter length is wrapped around the shaft 3 (inner tube 32) in a circumferential manner as shown in FIG. 17. Alternatively, by adjusting the diameter of the balloon 2 or the number of the wing 29, the balloon 2 can be configured so that the wrapping amount of the wing 29 can be reduced even at a part of the distal tapered part 24 and the proximal tapered part 22 closer to the straight tubular part 23 as shown in FIG. 17, or even so that almost no wing 29 is formed. By folding the balloon 2, the balloon 2 can be easily inserted into the body cavity.

As shown in FIG. 18, in the deflated state of the balloon 2, ends of the distal tapered part 24 and the proximal tapered part 22 on the straight tubular part 23 side in the longitudinal axis direction x of the balloon body 20 are termed as a position of 0% D₀ and the other ends are termed as a position of 100% D₁₀₀, and the balloon 2 preferably satisfies the at least one of the following (1) and (2):
(1) the tip part 61 of the protrusion part 60 in the entire extent of a section from a position of 20% D₂₀ to a position of 70% D₇₀ of the distal tapered part 24 is located on the first direction C₁ side or on the second direction C₂ side with respect to the straight line L_{d}, and the tip part 61 of the protrusion part 60 in a section from a position of 90% D₉₀ to the position of 100% D₁₀₀ of the distal tapered part 24 is not located on either the first direction C₁ side or the second direction C₂ side with respect to the straight line L_{d}; and
(2) the tip part 61 of the protrusion part 60 in the entire extent of a section from a position of 20% D₂₀ to a position of 70% D₇₀ of the proximal tapered part 22 is located on the first direction C₁ side or on the second direction C₂ side with respect to the straight line Lₚ, and the tip part 61 of the protrusion part 60 in a section from a position of 90% D₉₀ to the position of 100% D₁₀₀ of the proximal tapered part 22 is not located on either the first direction C₁ side or the second direction C₂ side with respect to the straight line Lₚ.

Since the section from the position of 90% D₉₀ to the position of 100% D₁₀₀ of the distal tapered part 24 and the proximal tapered part 22, which is farthest from the straight tapered part 23, is the tip side when the balloon 2 is moved forward or backward in the body cavity, straight incision into the body cavity wall with the tip side of the balloon 2 becomes available with the configuration in which the tip part 61 at the section of the protrusion part 60 in at least one of the distal tapered part 24 and the proximal tapered part 22 is not placed on either the first direction C₁ side or the second direction C₂ side with respect to the straight line L_{d} or the straight line Lₚ. In addition to such a configuration, the configuration where the tip part 61 of the protrusion part 60 in the entire extent of a section from the position of 20% D₂₀ to the position of 70% D₇₀ of at least one of the distal tapered part 24 and the proximal tapered part 22 is located on the first direction C₁ side or on the second direction C₂ side with respect to the straight line L_{d} or the straight line Lₚ allows the straight incision that has been made with the tip side of the balloon 2 to be extended obliquely by moving the balloon 2 forward or backward, or can make an incision in wide area with a single movement.

While FIG. 18 shows the embodiment that satisfies both the above conditions (1) and (2), either the distal tapered part 24 or the proximal tapered part 22 may satisfy either the above condition (1) or (2). From the viewpoint of making a straight incision with both tip sides of the balloon 2, the distal tapered part 24 and the proximal tapered part 22 preferably satisfy both the above (1) and (2). This allows the tip sides of the balloon 2 to make a straight incision, whether the balloon 2 is moved forward or backward.

As shown in FIG. 19, the balloon 2 in accordance with embodiments of the present invention preferably further satisfies at least one of the following (1) and (2) in the above-described embodiment:
(1) the distance from the straight line L_{d} to the tip part 61 of the protrusion part 60 at a position of 40% D₄₀ of the distal tapered part 24 is 1.2 times or longer than the distance from the straight line L_{d} to the tip part 61 of the protrusion part 60 at a position of 60% D₆₀ of the distal tapered part 24; and
(2) the distance from the straight line Lₚ to the tip part 61 of the protrusion part 60 at a position of 40% D₄₀ of the proximal tapered part 22 is 1.2 times or longer than the distance from the straight line Lₚ to the tip part 61 of the protrusion part 60 at a position of 60% D₆₀ of the proximal tapered part 22.

The distance from the straight line L_{d} or the straight line Lₚ to the tip part 61 of the protrusion part 60 at a position of 40% D₄₀ is preferably 1.5 times or longer than the distance from the straight line L_{d} or the straight line Lₚ to the tip part 61 of the protrusion part 60 at a position of 60% D₆₀, more preferably 2 times or longer. The distance from the straight line L_{d} or the straight line Lₚ to the tip part 61 of the protrusion part 60 at a position of 40% D₄₀ is preferably 10 times or shorter than the distance from the straight line L_{d} or the straight line Lₚ to the tip part 61 of the protrusion part 60 at a position of 60% D₆₀, more preferably 8 times or shorter, even more preferably 5 times or shorter. The above configuration allows the tip part 61 of the protrusion part 60 in at least one of the distal tapered part 24 and the proximal tapered part 22 to form more curved portion in the entire extent of the section from the position of 20% D₂₀ to the position of 70% D₇₀ in which the tip part 61 is located on the first direction C₁ side or the second direction C₂ side with respect to the straight line L_{d} or the straight line Lₚ, thereby allowing an oblique incision while the balloon 2 is moved forward or backward, or incision of wide area in a single movement.

As shown in FIG. 20, the balloon 2 preferably satisfies at least one of the following (1) and (2) in the deflated state:
(1) the tip part 61 of the protrusion part 60 in the entire extent of the section from the position of 20% D₂₀ to the position of 70% D₇₀ of the distal tapered part 24 is located inside or at the same position in the radial direction y of the balloon body 20 with respect to an imaginary curved surface C_{d} obtained by rotating the straight line L_{d} around a central axis 20C of the balloon body 20; and
(2) the tip part 61 of the protrusion part 60 in the entire extent of the section from the position of 20% D₂₀ to a position of 70% D₇₀ of the proximal tapered part 22 is located inside or at the same position in the radial direction y of the balloon body 20 with respect to an imaginary curved surface Cₚ obtained by rotating the straight line Lₚ around a central axis 20C of the balloon body 20.

This configuration where the tip part 61 of the protrusion part 60 of at least one of the distal tapered part 24 and the proximal tapered part 22 is located inside or at the same position in the radial direction y of the balloon body 20 with respect to the imaginary curved surface C_{d} or the imaginary curved surface Cₚ can reduce the diameter of the portion, allowing the balloon 2 to be easily inserted when moving forward or backward in the body cavity.

As shown in FIG. 20, the straight line L_{d} and the straight line Lₚ may be at an angle in the radial direction y with respect to the central axis 20C of the balloon body 20 (i.e., the longitudinal direction x). The imaginary curved surface C_{d} and the imaginary curved surface Cₚ in the embodiment where the straight line L_{d} and the straight line Lₚ are at an angle in the radial direction y with respect to the central axis 20C of the balloon body 20 are the side surface of a truncated cone, as shown in FIG. 20. When the diameter of the balloon 2 is large, or when the length of the protrusion part 60 at the straight tubular part 23 in the radial direction y is longer than the length of the protrusion part 60 at the distal tapered part 24 and the proximal tapered part 22 in the radial direction y in a cross section in the radial direction y, the straight line L_{d} or the straight line Lₚ is angled in the radial direction y with respect to the central axis 20C of the balloon body 20, and the imaginary curved surface C_{d} and the imaginary curved surface Cₚ are the side surface of a truncated cone the bottom surface of which is at the straight tubular part 23 side. In such an embodiment, the diameter of a distal side part and a proximal side part of the distal tapered part 24 and the proximal tapered part 22, respectively, can be kept small in the deflated state, and thus, the diameter of the portions that are tip sides when the balloon 2 is inserted into the body cavity and moved forward or backward is made small, allowing the balloon 2 to be easily inserted in the body cavity.

Although not shown in the figures, the straight line L_{d} and the straight line Lₚ may be parallel to the central axis 20C of the balloon body 20 (i.e., the longitudinal direction x). In the embodiment where the straight line L_{d} and the straight line Lₚ are parallel to the central axis 20C of the balloon body 20, the imaginary curved surface C_{d} and the imaginary curved surface Cₚ are the side surface of a cylinder. When the straight line L_{d} and the straight line Lₚ are parallel to the central axis 20C of the balloon body 20, the diameter of the straight tubular part 23 can be reduced in the deflated state of the balloon 2, and thus, the diameter of the straight tubular part 23 when the wing 29 formed by the deflation of the balloon 2 is wrapped around the shaft 3 also can be reduced, allowing easy insertion in the body cavity.

While FIG. 20 shows the embodiment where both the imaginary curved surface C_{d} and the imaginary curved surface Cₚ at the distal tapered part 24 and the proximal tapered part 22 are the side surface of a truncated cone, the imaginary curved surface C_{d} at the distal tapered part 24 may be the side surface of a cylinder and the imaginary curved surface Cₚ at the proximal tapered part 22 may be the side surface of a truncated cone, or vice versa.

While FIG. 20 shows the embodiment where both tapered parts in the entire extent of the section from the position of 20% D₂₀ to the position of 70% D₇₀ satisfy the above requirements (1) and (2), embodiments of the balloon 2 of the present invention include an embodiment where either the distal tapered part 24 or the proximal tapered part 22 satisfies the above requirement (1) or (2). From a viewpoint of improving insertion when the balloon 2 is inserted into the body cavity and moved forward to the lesion, the tip part 61 of the protrusion part 60 in the entire extent of the section from the position of 20% D₂₀ to the position of 70% D₇₀ of the distal tapered part 24 preferably placed inside or at the same position in the radial direction y of the balloon body 20 with respect to the imaginary curved surface C_{d}. This can make the diameter of the part that is at tip end side when the balloon 2 is inserted into the body cavity and moved forward small, allowing the balloon 2 to be more easily inserted in the body cavity.

As described above, in the embodiments of the balloon 2 of the present invention, the tip part 61 of the protrusion part 60 of at least one of the distal tapered part 24 and the proximal tapered part 22 is arranged curved on the first direction C₁ side or the second direction C₂ side in the circumferential direction z of the balloon body 20 independently with respect to the straight line L_{d} and the straight line Lₚ, respectively in the deflated state of the balloon 2, but as shown in FIG. 21, in the inflated state, the tip part 61 of the protrusion part 60 of the distal tapered part 24, the tip part 61 of the protrusion part 60 of the straight tubular part 23, and the tip part 61 of the protrusion part 60 of the proximal tapered part 22 preferably located at the same position in the circumferential direction z of the balloon body 20. This allows the balloon 2 to be capable of making a straight incision when the balloon is inflated at a lesion or the like after delivery, and in the deflated state, the balloon can make an oblique incision by moving forward or backward, or make an incision of wide area in a single movement.

As shown in FIG. 21, the protrusion part 60 of the distal tapered part 24, the protrusion part 60 of the straight tubular part 23, and the protrusion part 60 of the proximal tapered part 22 preferably extend continuously in the longitudinal axis direction x of the balloon body 20. The protrusion part 60 extending continuously in the longitudinal axis direction x of the balloon body 20 can improve the strength of the balloon 2 or easily further prevent overinflation of the balloon 2 when pressurized.

As shown in FIGs 3 to 5, and 15 to 17, the balloon body 20 preferably has a wing forming portion 28 that forms the wing 29 in the deflated state, and the protrusion part 60 is preferably located outside the wing forming portion 28. When the protrusion part 60 is located outside the wing forming portion 28, the protrusion part 60 do not interfere with the folding of the wing 29, allowing the balloon 2 to be easily folded and reducing the outer diameter of the balloon 2 in the folded state. In a more preferable embodiment, a plurality of the wing 29 is formed in the deflated state as shown in FIG. 4 and FIG. 5, and the protrusion part 60 is preferably arranged between each wing 29. This allows the protrusion part 60 to be protected by the wing 29 when the balloon 2 is folded as shown in FIG. 15 and FIG. 16, and can prevent damage to the protrusion part 60 or prevent the protrusion part 60 from acting on the body cavity wall in unintended places when the balloon 2 is folded and inserted into the body cavity. By adjusting the length of the wing 29 in the radial direction y by adjusting the diameter of the balloon 2 or the number of the wing 29, the extent to which the wing 29 covers the protrusion part 60 in the distal tapered part 24 and the proximal tapered part 22 can be adjusted as shown in FIG. 16 and FIG. 17. In other words, when the wing 29 is short enough not to cover the protrusion part 60 in the distal tapered part 24 and the proximal tapered part 22 at a part near the position of 0% D₀ close to the straight tubular part 23, the protrusion part 60 can be exposed from the wing 29 in most of the distal tapered part 24 and the proximal tapered part 22, and this exposed protrusion part 60 can be used to incise the stenosis while moving the balloon 2 forward or backward. Alternatively, the wing 29 can be made long enough to cover the protrusion part 60 beyond the position of 50% of the distal tapered part 24 and the proximal tapered part 22, in which case the portion of the protrusion part 60 exposed from the wing 29 can be made smaller, and thus, reducing the action of the protrusion part 60 when the balloon 2 is moved forward or backward. Thus, by adjusting the extent to which the wing 29 covers the protrusion part 60, it is possible to accommodate application to a variety of lesions.

While FIGs 4, 5, and 15 to 17 show the embodiment in which the number of the wing 29 is three, the number of the wing 29 is not particularly limited as long as the balloon 2 can be folded, and for example, the number of the wing 29 is preferably 2 or more, more preferably 3 or more, or may be 4 or more, or 5 or more. The lower limit of the number of the wing 29 in the above range can reduce the diameter of the balloon 2 while covering the protrusion part 60 when folded to improve insertion in the body cavity. The number of the wing 29 is preferably 10 or less, more preferably 8 or less, and even more preferably 6 or less. When the upper limit of the number of the wing 29 is within the above range, even the balloon 2 with a large diameter can be easily folded. By setting the number of the wing 29 in the above range, the size of the portion of the protrusion part 60 covered by the wing 29 in the distal tapered part 24 and the proximal tapered part 22 can be adjusted.

Materials forming the balloon body 20 include, for example, polyolefin-based resin such as polyethylene, polypropylene, ethylene-propylene copolymer; polyester-based resin such as polyethylene terephthalate and polyester elastomer; polyurethane-based resin such as polyurethane and polyurethane elastomer; polyphenylene sulfide-based resin; polyamide-based resin such as polyamide and polyamide elastomer; fluorine-based resin; silicone-based resin; and natural rubber such as latex rubber. Only one of these may be used, or two or more may be used in combination. Of these, polyamide-based resin, polyester-based resin, and polyurethane-based resin are preferably used. In particular, elastomer resin is preferably used from the viewpoint of thinning and flexibility of the balloon body 20. For example, among polyamide-based resins, nylon 12, nylon 11, and the like are suitable for the resin forming the balloon body 20, and more preferably nylon 12 because it is relatively easy to mold when blow molding. Polyamide elastomers such as polyether ester amide elastomer and polyamide ether elastomer are also preferred in terms of thinning and flexibility of the balloon body 20. Of these, polyether ester amide elastomer is preferred in terms of high yield strength and good dimensional stability of the balloon body 20.

The protrusion part 60 is preferably made of the same material as the balloon body 20. When the protrusion part 60 is made of the same material as the balloon body 20, the protrusion part 60 is less likely to damage the outer surface of the balloon body 20 while maintaining the flexibility of the balloon 2. The balloon body 20 and the protrusion part 60 are integrally molded. This can prevent the protrusion part 60 from falling off from the balloon body 20.

Materials forming the shaft 3 include, for example, polyamide-based resin, polyester-based resin, polyurethane-based resin, polyolefin-based resin, fluorine-based resin, polyvinyl chloride-based resin, silicone-based resin, and natural rubber. Only one of these may be used, or two or more may be used in combination. Of these, the material forming the shaft 3 is preferably at least one of polyamide-based resin, polyolefin-based resin, and fluorine-based resin. This can improve surface slipperiness of the shaft 3 and improve the insertion of the balloon catheter 1 into the body cavity.

The balloon 2 and the shaft 3 may be joined by adhesive bonding, welding, or by attaching a ring-shaped member at the point where the end of the balloon 2 and the shaft 3 overlap to swage them. Of these, the balloon 2 and the shaft 3 are preferably joined by welding. By welding the balloon 2 and the shaft 3, the bond between the balloon 2 and the shaft 3 is difficult to be released even when the balloon 2 is repeatedly inflated and deflated, easily increasing the strength of the bond between the balloon 2 and the shaft 3.

As shown in FIG. 1, the balloon catheter 1 may be provided with a hub 4 at a proximal side of the shaft 3, and the hub 4 may be provided with a fluid inlet 7 that is connected to the flow channel of the fluid supplied to the interior of the balloon 2. In addition, the hub 4 preferably has a guidewire insertion port 5 that is connected to the guidewire insertion channel. The balloon catheter 1 having the hub 4 provided with the fluid inlet 7 and the guidewire insertion port 5 can facilitate the operation of supplying fluid inside the balloon 2 to inflate and deflate the balloon 2 and delivering the balloon catheter 1 to a lesion site along a guidewire. The balloon 2 in accordance with embodiments of the present invention is applicable not only the so-called over-the-wire balloon catheter in which the guidewire is inserted over the distal to the proximal side of the shaft 3 as shown in FIG. 1, but also applicable to a so-called rapid exchange balloon catheter in which the guidewire is inserted from the distal side to the midway of the proximal side of the shaft.

The shaft 3 and the hub 4 may be joined by, for example, adhesive bonding or welding. Of these, the shaft 3 and the hub 4 are preferably joined by adhesive bonding. The adhesive bonding of the shaft 3 and the hub 4 can increase the bonding strength of the shaft 3 and the hub 4 to increase durability of the balloon catheter 1 when the materials forming the shaft 3 and the hub 4 are different, for example, in a case where the shaft 3 is made of material having high flexibility and the hub 4 is made of material having high stiffness.

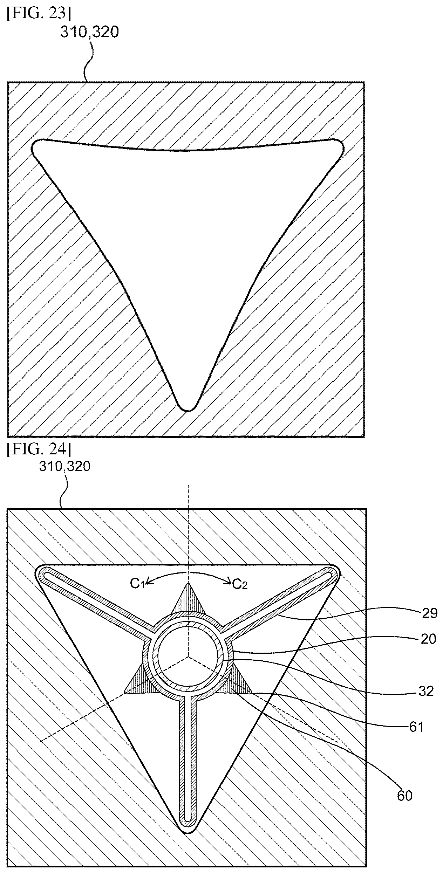

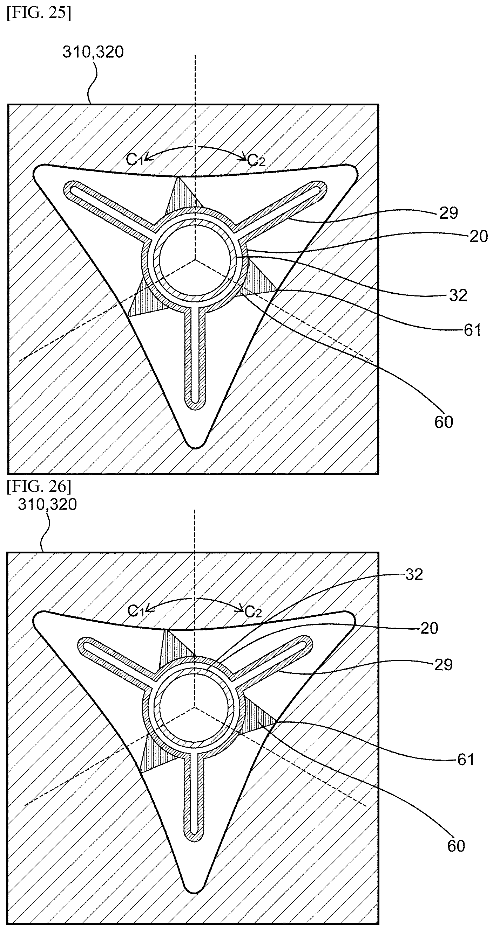

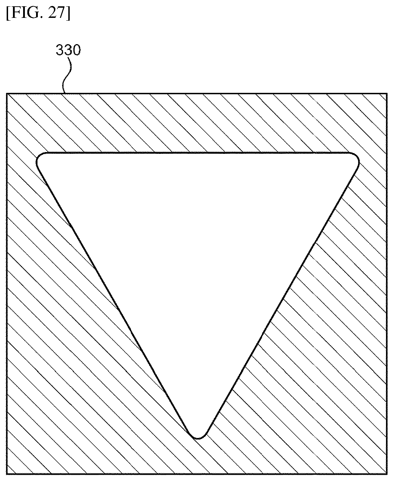

The present invention also provides a method for producing the balloon 2 for balloon catheter 1 in accordance with embodiments of the present invention. The method for producing the balloon 2 in accordance with embodiments of the present invention will be described referring to FIGs 22 to 27. FIG. 22 is a perspective view of a parison before expansion in accordance with one embodiment of the present invention, showing a configuration having a lumen and a thick-walled portion. FIG. 23 is a cross-sectional view in the radial direction of a first tubular member and a second tubular member of a producing method in accordance with one embodiment of the present invention. FIG. 24 is a radial cross-sectional view in a process of placing the distal tapered part in the first tubular member or a process of placing the proximal tapered part in the second tubular member in the producing method in accordance with one embodiment of the present invention, showing a radial cross-sectional view of a part in which the proximal end of the distal tapered part or the distal end of the proximal tapered part is placed in the first tubular member or the second tubular member, respectively. FIG. 25 is a radial cross-sectional view of the first tubular member or the second tubular member shown in FIG. 24 at different position in the longitudinal axis direction, showing a radial cross-sectional view of a position at which a part where the tip part of the protrusion part of the distal tapered part is located on the first direction side in the circumferential direction of the balloon body with respect to the straight line L_{d} is placed, or a part where the tip part of the protrusion part of the proximal tapered part is located on the first direction side in the circumferential direction of the balloon body with respect to the straight line Lₚ. FIG. 26 is a radial cross-sectional view of the first tubular member or the second tubular member shown in FIG. 24 at still different position in the longitudinal axis direction, showing a radial cross-sectional view of a position at which a part where the tip part of the protrusion part of the distal tapered part is located further on the first direction side in the circumferential direction of the balloon body with respect to the straight line L_{d} is placed, or a part where the tip part of the protrusion part of the proximal tapered part is located further on the first direction side in the circumferential direction of the balloon body with respect to the straight line Lₚ. The dashed lines in FIGs 24 to 26 represent the circumferential position of the tip part of the protrusion part at the proximal end of the distal tapered part or the circumferential position of the tip part of the protrusion part at the distal end of the proximal tapered part, which correspond to the base point of the straight line L_{d} or Lₚ. FIG. 27 is a radial cross-sectional view of a third tubular member of the producing method in accordance with one embodiment of the present invention.

A method for producing the balloon 2 in accordance with an embodiment of the present invention has a process of preparing a first tubular member 310, a second tubular member 320, and a third tubular member 330, each of which has an inner space extending in a longitudinal axis direction; a process of preparing a balloon for a balloon catheter having the balloon body 20 having an outer surface and an inner surface, the balloon body 20 having a straight tubular part 23, the distal tapered part 24 located distal to the straight tubular part 23, and the proximal tapered part 22 located proximal to the straight tubular part 23, and the distal tapered part 24, the straight tubular part 23, and the proximal tapered part 22 having the protrusion part 60 that projects outwardly in the radial direction y from the outer surface of the balloon body 20 and extends in the longitudinal axis direction x of the balloon body 20; and a process of placing the distal tapered part 24 in the first tubular member 310, placing the proximal tapered part 22 in the second tubular member 320, and placing the straight tubular part 23 in the third tubular member 330 in the deflated state of the balloon; and the method satisfies at least one of the following (1) and (2):
(1) at least a part of the protrusion part 60 of the distal tapered part 24 is in contact with an inner surface of the first tubular member 310 in the placing process; and
(2) at least a part of the protrusion part 60 of the proximal tapered part 22 is in contact with an inner surface of the second tubular member 320 in the placing process.

Placing so that a part of the protrusion part 60 of the distal tapered part 24 is in contact with the inner surface of the first tubular member 310 allows the protrusion part 60 to be moved in the circumferential direction z of the balloon body 20. As a result, the tip part 61 of the protrusion part 60 of the distal tapered part 24 can be placed to curve toward the first direction C₁ side or the second direction C₂ side in the circumferential direction z of the balloon body 20 in the deflated state.

Placing so that a part of the protrusion part 60 of the proximal tapered part 22 is in contact with the inner surface of the second tubular member 320 allows the protrusion part 60 to be moved in the circumferential direction z of the balloon body 20. As a result, the tip part 61 of the protrusion part 60 of the proximal tapered part 22 can be placed to curve toward the first direction C₁ side or the second direction C₂ side in the circumferential direction z of the balloon body 20 in the deflated state.

To move the tip part 61 of the protrusion part 60 of the distal tapered part 24 in the circumferential direction z of the balloon body 20 in the deflated state, the above process (1) can be performed: to move the tip part 61 of the protrusion part 60 of the proximal tapered part 22 in the circumferential direction z of the balloon body 20 in the deflated state, the above process (2) can be performed: to move the tip part 61 of the protrusion part 60 of both the distal tapered part 24 and the proximal tapered part 22 in the circumferential direction z of the balloon body 20 in the deflated state, both the above (1) and (2) can be performed.

In the process of preparing the balloon, for example, the balloon can be prepared by placing a tubular parison 200 made of a resin as shown in FIG. 22 in a mold having a groove, followed by biaxially stretch-blow molding. The protrusion part 60 can be formed, for example, by inserting the parison 200 into the inner cavity of the mold such that a thick-walled portion 220 of the parison 200 is inserted into the groove of the mold, and then expanding the parison 200 by introducing fluid into a lumen 210 of the parison 200. When the protrusion part 60 is not formed on the distal sleeve part 25 or the proximal sleeve part 21 or the inner protrusion part is formed, for example, the balloon 2 can be produced by pressing the thick-walled portion 220 of the parison 200 against the part of the mold other than the groove, and then expanding the parison 200 by introducing fluid into the lumen 210 of the parison 200. The materials forming the parison 200 can be referred to the description of the materials forming the balloon body 20.

The first tubular member 310 in the case where the above process (1) of the producing method in accordance with the present invention, as shown in FIG. 23, has an inner space extending in the longitudinal direction x, and the cross-sectional shape in the radial direction y of the space is preferably different in the longitudinal direction x. In other words, the shape of the above-described space is different depending on the distance at which the tip part 61 of the protrusion part 60 of the distal tapered part 24 is moved in the circumferential direction z of the balloon body 20 in the deflated state. This allows the position where the inner surface of the first tubular member 310 and at least a part of the protrusion part 60 of the distal tapered part 24 is in contact with each other to be changed, and the distance at which the tip part 60 of the protrusion part 60 of the distal tapered part 24 is moved in the circumferential direction z of the balloon body 20 can be controlled while preventing the tip part 61 of the protrusion part 60 of the distal tapered part 24 from moving in the radial direction y of the balloon body 20 in the deflated state.

Examples of the shape of the space of the first tubular member 310 in a cross section in the radial direction y are described. The space of the first tubular member 310 at which the distal and proximal ends of the distal tapered part 24, i.e., at which the tip part 61 of the protrusion part 60 of the distal tapered part 24 at the base end and tip end of the straight line L_{d}, preferably has a shape that does not come into contact with the protrusion part 60, and for example, the space of the first tubular member 310 at a position at which the proximal end of the distal tapered part 24 is placed may be the shape shown in FIG. 24. When the moving of the tip part 61 of the protrusion part 60 of the distal tapered part 24 in the radial direction y of the balloon body 20 is to be prevented, the space of the first tubular member 310 at a position at which the portion of the tip part 61 of the protrusion part 60 of the distal tapered part 24 is placed on the first direction C₁ side in the circumferential direction z of the balloon body 20 with respect to the straight line L_{d} preferably has a shape that is partially in contact with the protrusion part 60 as shown in FIG. 25. The space of the first tubular member 310 at a position at which the portion of the tip part 61 of the protrusion part 60 of the distal tapered part 24 is placed further on the first direction C₁ side in the circumferential direction with respect to the straight line L_{d} preferably has a shape that is partially in contact with the protrusion part 60 as shown in FIG. 26. Thus, the change in shape of the space, as exemplified in FIGs 24 to 26, allows the distance at which the tip part 61 of the protrusion part 60 of the distal tapered part 24 is moved in the circumferential direction z of the balloon body 20 to be adjusted while preventing the tip part 61 of the protrusion part 60 of the distal tapered part 24 from moving in the radial direction y of the balloon body 20 in the deflated state. Alternatively, although not shown in the figures, adjusting the cross-sectional shape of the space in the radial direction y of the first tubular member 310 can bring the inner surface of the first tubular member 310 into contact with a part of the protrusion part 60 while allowing the tip part 61 of the protrusion part 60 to move in the radial direction y, and thus, the tip part 61 of the protrusion part 60 can be moved in the circumferential direction z of the balloon body 20 allowing movement in the radial direction y, and the distance of movement in the circumferential direction z along the longitudinal direction x can be adjusted. The shape of the space of the first tubular member 310 is not limited by the examples shown in FIGs 24 to 26, and may be any shape as long as the distance at which the tip part 61 of the protrusion part 60 of the distal tapered part 24 is moved in the circumferential direction z of the balloon body 20 while preventing or allowing the movement in the radial direction y in the deflated state can be adjusted.

The cross-sectional shape in the radial direction y of the first tubular member 310 and the second tubular member 320 preferably varies continuously in the longitudinal axis direction x. This allows the tip part 61 of the protrusion part 60 of the distal tapered part 24 and the tip part 61 of the protrusion part 60 of the proximal tapered part 22 to be moved in the circumferential direction z of the balloon body 20 continuously in the longitudinal direction x in the deflated state.

The third tubular member 330 has an inner space extending in the longitudinal direction x. The shape of the space of the third tubular member 330 in a cross section in the radial direction y may be the same as or different from the shape of the space of the first tubular member 310 at the position where the proximal end of the distal tapered part 24 is placed as shown in FIG. 27, and the area of the space of the third tubular member 330 in a cross section in the radial direction y is preferably larger than the area of the space of the first tubular member 310. This can prevent the protrusion part 60 of the straight tubular part 23, which is placed in the space of the third tubular member 330, from contacting the inner surface of the third tubular member 330, so that the inner surface of the third tubular member 330 does not interfere with the protrusion part 60 of the straight tubular part 23.

In the case where the process (2) is also performed in addition to the above-described process (1), the second tubular member 320 preferably has the same configuration as the above-described first tubular member 310. That is, the shape of the space of the second tubular member 320 in a cross section in the radial direction y at the position where the distal end of the proximal tapered part 22 is placed may be the shape as shown in FIG. 24, the shape of the second tubular member 320 at the position where a part in which the tip part 61 of the protrusion part 60 of the proximal tapered part 22 is located on the first direction C₁ side in the circumferential direction with respect to the straight line L_{d} is placed may be the shape as shown in FIG. 25, and the shape of the second tubular member 320 at the position where a part in which the tip part 61 of the protrusion part 60 of the proximal tapered part 22 is located further on the first direction C₁ side in the circumferential direction with respect to the straight line L_{d} is placed may be the shape as shown in FIG. 26. Similar to the first tubular member 310, the shape of the space of the second tubular member 320 in a cross section in the radial direction y is not limited to these examples. The second tubular member 320 having the above-described configuration can change the position where the inner surface of the second tubular member 320 and a part of the protrusion part 60 of the proximal tapered part 22 are in contact with each other, and can adjust the distance at which the tip part 61 of the protrusion part 60 of the proximal tapered part 22 is moved in the circumferential direction z of the balloon body 20 while preventing or allowing the movement in the radial direction y of the balloon body 20 in the deflated state.

In the case where only the above process (1) is performed and the process (2) is not performed, the second tubular member 320 preferably has the same configuration as the above-described third tubular member 330. This can prevent the protrusion part 60 of the proximal tapered part 22, which is placed in the space of the second tubular member 320, from contacting the inner surface of the second tubular member 320, so that inner surface of the second tubular member 320 does not interfere with the protrusion part 60 of the proximal tapered part 22.

In the case where only the above process (2) is performed and the process (1) is not performed, the first tubular member 310 preferably has the same configuration as the above-described third tubular member 330. This can prevent the protrusion part 60 of the distal tapered part 24, which is placed in the space of the first tubular member 310, from contacting the inner surface of the first tubular member 310, so that the inner surface of the first tubular member 310 does not interfere with the protrusion part 60 of the distal tapered part 24.

The length of the first tubular member 310 in the longitudinal direction x, the length of the second tubular member 320 in the longitudinal direction x, and the length of the third tubular member 330 in the longitudinal direction x are preferably almost the same as the length of the distal tapered part 24 in the longitudinal direction x, the length of the proximal tapered part 22 in the longitudinal direction x, and the length of the straight tubular part 23 in the longitudinal direction x, respectively.

In the above-described placing process, the first tubular member 310, the third tubular member 330, and the second tubular member 320 are preferably lined up in this order in the longitudinal direction x so that each center of the space in the cross section of the radial direction y is coincident, and the balloon 2 is preferably inserted into the space from the second tubular member 320 side. This allows the distal tapered part 24 to be placed in the first tubular member 310, the straight tubular part 23 to be placed in the third tubular member 330, and the proximal tapered part 22 to be placed in the second tubular member 320.

By performing the above-described placing process to determine the placement of the tip part 61 of the protrusion part 60, the placement of the tip part 61 of the protrusion part 60 in the distal tapered part 24 and/or the proximal tapered part 22 can be deformed. Subsequently, the balloon 2 can be folded by hand or using various folding machines. When the protrusion part 60 is disposed outside the wing forming portion 28, the balloon 2 is preferably folded so that the wing 29 covers the protrusion part 60. By folding the balloon 2 so as not to disrupt the deformed arrangement of the tip part 61 of the protrusion part 60, it is possible to obtain the balloon 2 in which the tip part 61 of the protrusion part 60 is curved in the circumferential direction z of the balloon body 20 in the folded state.

Materials forming that make up the first tubular member 310, the second tubular member 320, and the third tubular member 330 include, for example, synthetic resins such as polycarbonate-based resin, polyacetal-based resin, and fluorine-based resin; and metals such as iron, copper, and stainless steel.

### DESCRIPTION OF REFERENCE SIGNS

1: balloon catheter
2: balloon
3: shaft
4: hub
5: guidewire insertion port
7: fluid inlet
20: balloon body
20C: central axis of balloon body
21: proximal sleeve part
22: proximal tapered part
23: straight tubular part
24: distal tapered part
25: distal sleeve part
28: wing forming portion
29: wing
31: outer tube
32: inner tube
60: protrusion part
61: tip part
200: parison
210: lumen of parison
220: thick-walled portion of parison
310: first tubular member
320: second tubular member
330: third tubular member
L_{d}: straight line connecting tip part at D₀ and tip part at D₁₀₀ of distal tapered part
Lₚ: straight line connecting tip part at D₀ and tip part at D₁₀₀ of proximal tapered part
C₁: first direction
C₂: second direction
D₀: position of 0%
D₂₀: position of 20%
D₄₀: position of 40%
D₆₀: position of 60%
D₇₀: position of 70%
D₉₀: position of 90%
D₁₀₀: position of 100%
C_{d}: imaginary curved surface obtained by rotating L_{d} around central axis of balloon body
Cₚ: imaginary curved surface obtained by rotating Lₚ around central axis of balloon body
x: longitudinal axis direction
y: radial direction
z: circumferential direction

## Claims

1. A balloon (2) for a balloon catheter (1) comprising a balloon body (20) having an outer surface and an inner surface, wherein
the balloon body has a straight tubular part (23), a distal tapered part (24) located distal to the straight tubular part, and a proximal tapered part (22) located proximal to the straight tubular part,
the distal tapered part, the straight tubular part, and the proximal tapered part have a protrusion part (66) that projects outwardly in a radial direction (y) from the outer surface of the balloon body and extends in a longitudinal axis direction (x) of the balloon body,
the protrusion part has a tip part (61) in a cross section in the radial direction of the balloon body,
the balloon body and the protrusion part are integrally molded;
the balloon satisfies at least one of the following (1) and (2):
(1) in a deflated state of the balloon for a balloon catheter, the tip part of the protrusion part of the distal tapered part is located on a first direction (C₁) side and not on a second direction (C₂) side, or located on the second direction side and not on the first direction side, in a circumferential direction (z) of the balloon body with respect to a straight line L_{d} connecting the tip part at a proximal end of the distal tapered part and the tip part at a distal end of the distal tapered part; and
(2) in a deflated state of the balloon for a balloon catheter, the tip part of the protrusion part of the proximal tapered part is located on a first direction (C₁) side and not on a second direction (C₂) side, or located on the second direction side and not on the first direction side, in a circumferential direction (z) of the balloon body with respect to a straight line Lₚ connecting the tip part at a distal end of the proximal tapered part and the tip part at a proximal end of the proximal tapered part;
**characterized in that** the tip part of the protrusion part of at least one of the distal tapered part and the proximal tapered part is arranged curved on the first direction side or the second direction side in the circumferential direction of the balloon body independently with respect to the straight line L_{d} and the straight line Lₚ, respectively in the deflated state of the balloon.

2. The balloon for a balloon catheter according to claim 1, wherein the balloon for a balloon catheter is folded in the deflated state of the balloon for a balloon catheter.

3. The balloon for a balloon catheter according to claim 1 or 2, wherein in the deflated state of the balloon for a balloon catheter, ends of the distal tapered part and the proximal tapered part on the straight tubular part side are termed as a position of 0% and the other ends of the distal tapered part and the proximal tapered part are termed as a position of 100% in the longitudinal axis direction of the balloon body, and the balloon satisfies at least one of the following (1) and (2):
(1) the tip part of the protrusion part in the entire extent of a section from a position of 20% to a position of 70% of the distal tapered part is located on the first direction side or on the second direction side with respect to the straight line L_{d}, and the tip part of the protrusion part in a section from a position of 90% to the position of 100% of the distal tapered part is not located on either the first direction side or the second direction side with respect to the straight line L_{d}; and
(2) the tip part of the protrusion part in the entire extent of a section from a position of 20% to a position of 70% of the proximal tapered part is located on the first direction side or on the second direction side with respect to the straight line Lₚ, and the tip part of the protrusion part in a section from a position of 90% to the position of 100% of the proximal tapered part is not located on either the first direction side or the second direction side with respect to the straight line Lₚ.

4. The balloon for a balloon catheter according to claim 3, satisfying at least one of the following (1) and (2):
(1) a distance from the straight line L_{d} to the tip part of the protrusion part at a position of 40% of the distal tapered part is 1.2 times or longer than a distance from the straight line L_{d} to the tip part of the protrusion part at a position of 60% of the distal tapered part; and
(2) a distance from the straight line Lₚ to the tip part of the protrusion part at a position of 40% of the proximal tapered part is 1.2 times or longer than a distance from the straight line Lₚ to the tip part of the protrusion part at a position of 60% of the proximal tapered part.

5. The balloon for a balloon catheter according to any one of claims 1 to 4, satisfying at least one of the following (1) and (2) in the deflated state of the balloon for a balloon catheter:
(1) the tip part of the protrusion part in the entire extent of a section from a position of 20% to a position of 70% of the distal tapered part is located inside or at the same position in the radial direction of the balloon body with respect to an imaginary curved surface obtained by rotating the straight line L_{d} around a central axis of the balloon body; and
(2) the tip part of the protrusion part in the entire extent of a section from a position of 20% to a position of 70% of the proximal tapered part is located inside or at the same position in the radial direction of the balloon body with respect to an imaginary curved surface obtained by rotating the straight line Lₚ around a central axis of the balloon body.

6. The balloon for a balloon catheter according to any one of claims 1 to 5, wherein the tip part of the protrusion part of the distal tapered part, the tip part of the protrusion part of the straight tubular part, and the tip part of the protrusion part of the proximal tapered part are located at the same position in the circumferential direction of the balloon body in an inflated state of the balloon for a balloon catheter.

7. The balloon for a balloon catheter according to any one of claims 1 to 6, wherein the balloon body has a wing forming portion (28) that forms a wing (29) in the deflated state, and the protrusion part is located outside the wing forming portion.

8. The balloon for a balloon catheter according to any one of claims 1 to 7, wherein the protrusion part of the distal tapered part, the protrusion part of the straight tubular part, and the protrusion part of the proximal tapered part extend continuously in the longitudinal axis direction of the balloon body.

9. The balloon for a balloon catheter according to any one of claims 1 to 8, wherein the protrusion part is composed of the same material as the balloon body.

10. A method for producing the balloon for a balloon catheter according to any one of claims 1 to 9, comprising:
preparing a first tubular member (310), a second tubular member (320), and a third tubular member (330), each of which has an inner space extending in a longitudinal axis direction (x);
preparing a balloon (2) for a balloon catheter (1) having a balloon body (20) having an outer surface and an inner surface, the balloon body having a straight tubular part (23), a distal tapered part (24) located distal to the straight tubular part, and a proximal tapered part (22) located proximal to the straight tubular part, and the distal tapered part, the straight tubular part, and the proximal tapered part having a protrusion part (60) that projects outwardly in a radial direction from the outer surface of the balloon body and extends in a longitudinal axis direction of the balloon body; and
placing the distal tapered part in the first tubular member, placing the proximal tapered part in the second tubular member, and placing the straight tubular part in the third tubular member in the deflated state of the balloon for a balloon catheter, wherein
the method satisfies at least one of the following (1) and (2):
(1) at least a part of the protrusion part of the distal tapered part is in contact with an inner surface of the first tubular member in the placing process; and
(2) at least a part of the protrusion part of the proximal tapered part is in contact with an inner surface of the second tubular member in the placing process.

## Patentansprüche

1. Ballon (2) für einen Ballonkatheter (1), umfassend einen Ballonkörper (20), welcher eine Außenfläche und eine Innenfläche aufweist, wobei
der Ballonkörper einen geraden rohrförmigen Abschnitt (23), einen distalen konischen Abschnitt (24), welcher distal zu dem geraden rohrförmigen Abschnitt angeordnet ist, und einen proximalen konischen Abschnitt (22), welcher proximal zu dem geraden rohrförmigen Abschnitt angeordnet ist, aufweist,
der distale konische Abschnitt, der gerade rohrförmige Abschnitt und der proximale konische Abschnitt einen Vorsprungabschnitt (66) aufweisen, welcher in einer radialen Richtung (y) von der Außenfläche des Ballonkörpers nach außen ragt und sich in einer Longitudinalachsenrichtung (x) des Ballonkörpers erstreckt,
der Vorsprungabschnitt in einem Querschnitt in der radialen Richtung des Ballonkörpers einen Spitzenabschnitt (61) aufweist,
der Ballonkörper und der Vorsprungabschnitt einstückig geformt sind;
der Ballon wenigstens eine der folgenden Bedingungen (1) und (2) erfüllt:
(1) in einem entleerten Zustand des Ballons für einen Ballonkatheter ist der Spitzenabschnitt des Vorsprungabschnitts des distalen konischen Abschnitts in einer Umfangsrichtung (z) des Ballonkörpers bezüglich einer Geraden Ld, welche den Spitzenabschnitt an einem proximalen Ende des distalen konischen Abschnitts und den Spitzenabschnitt an einem distalen Ende des distalen konischen Abschnitts verbindet, auf einer Seite der ersten Richtung (C1) und nicht auf einer Seite der zweiten Richtung (C2) angeordnet oder auf der Seite der zweiten Richtung und nicht auf der Seite der ersten Richtung angeordnet, und
(2) in einem entleerten Zustand des Ballons für einen Ballonkatheter ist der Spitzenabschnitt des Vorsprungabschnitts des proximalen konischen Abschnitts in einer Umfangsrichtung (z) des Ballonkörpers bezüglich einer Geraden Lp, welche den Spitzenabschnitt an einem distalen Ende des proximalen konischen Abschnitts und den Spitzenabschnitt an einem proximalen Ende des proximalen konischen Abschnitts verbindet, auf einer Seite der ersten Richtung (C1) und nicht auf einer Seite der zweiten Richtung (C2) angeordnet oder auf der Seite der zweiten Richtung und nicht auf der Seite der ersten Richtung angeordnet;
**dadurch gekennzeichnet, dass** der Spitzenabschnitt des Vorsprungabschnitts wenigstens eines des distalen konischen Abschnitts und des proximalen konischen Abschnitts in der Umfangsrichtung des Ballonkörpers unabhängig bezüglich der Geraden Ld bzw. der Geraden Lp in dem entleerten Zustand des Ballons auf der Seite der ersten Richtung oder der Seite der zweiten Richtung gekrümmt angeordnet ist.

2. Ballon für einen Ballonkatheter nach Anspruch 1, wobei der Ballon für einen Ballonkatheter in dem entleerten Zustand des Ballons für einen Ballonkatheter gefaltet ist.

3. Ballon für einen Ballonkatheter nach Anspruch 1 oder 2, wobei in dem entleerten Zustand des Ballons für einen Ballonkatheter Enden des distalen konischen Abschnitts und des proximalen konischen Abschnitts auf der Seite des geraden rohrförmigen Abschnitts als eine Position von 0 % bezeichnet werden und die anderen Enden des distalen konischen Abschnitts und des proximalen konischen Abschnitts als eine Position von 100 % in der Longitudinalachsenrichtung des Ballonkörpers bezeichnet werden, und der Ballon wenigstens eine der folgenden Bedingungen (1) und (2) erfüllt:
(1) der Spitzenabschnitt des Vorsprungabschnitts in der gesamten Ausdehnung eines Abschnitts von einer Position von 20 % bis zu einer Position von 70 % des distalen konischen Abschnitts ist bezüglich der Geraden Ld auf der Seite der ersten Richtung oder auf der Seite der zweiten Richtung angeordnet; und der Spitzenabschnitt des Vorsprungabschnitts in einem Abschnitt von einer Position von 90 % bis zu der Position von 100 % des distalen konischen Abschnitts ist bezüglich der Geraden Ld weder auf der Seite der ersten Richtung noch auf der Seite der zweiten Richtung angeordnet; und
(2) der Spitzenabschnitt des Vorsprungabschnitts in der gesamten Ausdehnung eines Abschnitts von einer Position von 20 % bis zu einer Position von 70 % des proximalen konischen Abschnitts ist bezüglich der Geraden Lp auf der Seite der ersten Richtung oder auf der Seite der zweiten Richtung angeordnet; und der Spitzenabschnitt des Vorsprungabschnitts in einem Abschnitt von einer Position von 90 % bis zu der Position von 100 % des proximalen konischen Abschnitts ist bezüglich der Geraden Lp weder auf der Seite der ersten Richtung noch auf der Seite der zweiten Richtung angeordnet.

4. Ballon für einen Ballonkatheter nach Anspruch 3, welcher wenigstens eine der folgenden Bedingungen (1) und (2) erfüllt:
(1) ein Abstand von der Geraden Ld zu dem Spitzenabschnitt des Vorsprungabschnitts an einer Position von 40 % des distalen konischen Abschnitts beträgt das 1,2-Fache oder mehr eines Abstands von der Geraden Ld zu dem Spitzenabschnitt des Vorsprungabschnitts an einer Position von 60 % des distalen konischen Abschnitts; und
(2) ein Abstand von der Geraden Lp zu dem Spitzenabschnitt des Vorsprungabschnitts an einer Position von 40 % des proximalen konischen Abschnitts beträgt das 1,2-Fache oder mehr eines Abstands von der Geraden Lp zu dem Spitzenabschnitt des Vorsprungabschnitts an einer Position von 60 % des proximalen konischen Abschnitts.

5. Ballon für einen Ballonkatheter nach einem der Ansprüche 1 bis 4, welcher wenigstens eine der folgenden Bedingungen (1) und (2) in dem entleerten Zustand des Ballons für einen Ballonkatheter erfüllt:
(1) der Spitzenabschnitt des Vorsprungabschnitts in der gesamten Ausdehnung eines Abschnitts von einer Position von 20 % bis zu einer Position von 70 % des distalen konischen Abschnitts ist in der radialen Richtung des Ballonkörpers bezüglich einer imaginären gekrümmten Fläche, welche durch Rotieren der Geraden Ld um eine Mittelachse des Ballonkörpers erhalten ist, innerhalb oder an der gleichen Position angeordnet; und
(2) der Spitzenabschnitt des Vorsprungabschnitts in der gesamten Ausdehnung eines Abschnitts von einer Position von 20 % bis zu einer Position von 70 % des proximalen konischen Abschnitts ist in der radialen Richtung des Ballonkörpers bezüglich einer imaginären gekrümmten Fläche, welche durch Rotieren der Geraden Lp um eine Mittelachse des Ballonkörpers erhalten ist, innerhalb oder an der gleichen Position angeordnet.

6. Ballon für einen Ballonkatheter nach einem der Ansprüche 1 bis 5, wobei der Spitzenabschnitt des Vorsprungabschnitts des distalen konischen Abschnitts, der Spitzenabschnitt des Vorsprungabschnitts des geraden rohrförmigen Abschnitts und der Spitzenabschnitt des Vorsprungabschnitts des proximalen konischen Abschnitts in einem aufgeblasenen Zustand des Ballons für einen Ballonkatheter in der Umfangsrichtung des Ballonkörpers an der gleichen Position angeordnet sind.

7. Ballon für einen Ballonkatheter nach einem der Ansprüche 1 bis 6, wobei der Ballonkörper einen Flügelbildungsabschnitt (28) aufweist, welcher in dem entleerten Zustand einen Flügel (29) bildet, und der Vorsprungabschnitt außerhalb des Flügelbildungsabschnitts angeordnet ist.

8. Ballon für einen Ballonkatheter nach einem der Ansprüche 1 bis 7, wobei der Vorsprungabschnitt des distalen konischen Abschnitts, der Vorsprungabschnitt des geraden rohrförmigen Abschnitts und der Vorsprungabschnitt des proximalen konischen Abschnitts sich in der Longitudinalachsenrichtung des Ballonkörpers kontinuierlich erstrecken.

9. Ballon für einen Ballonkatheter nach einem der Ansprüche 1 bis 8, wobei der Vorsprungabschnitt aus dem gleichen Material wie der Ballonkörper besteht.

10. Verfahren zur Herstellung des Ballons für einen Ballonkatheter nach einem der Ansprüche 1 bis 9, umfassend:
Bereitstellen eines ersten rohrförmigen Elements (310), eines zweiten rohrförmigen Elements (320) und eines dritten rohrförmigen Elements (330), von denen jedes einen sich in einer Longitudinalachsenrichtung (x) erstreckenden Innenraum aufweist;
Bereitstellen eines Ballons (2) für einen Ballonkatheter (1) mit einem Ballonkörper (20), welcher eine Außenfläche und eine Innenfläche aufweist, wobei der Ballonkörper einen geraden rohrförmigen Abschnitt (23), einen distalen konischen Abschnitt (24), welcher distal zu dem geraden rohrförmigen Abschnitt angeordnet ist, und einen proximalen konischen Abschnitt (22), welcher proximal zu dem geraden rohrförmigen Abschnitt angeordnet ist, aufweist, und der distale konische Abschnitt, der gerade rohrförmige Abschnitt und der proximale konische Abschnitt einen Vorsprungabschnitt (60) aufweisen, welcher in einer radialen Richtung von der Außenfläche des Ballonkörpers nach außen ragt und sich in einer Longitudinalachsenrichtung des Ballonkörpers erstreckt; und
Einbringen des distalen konischen Abschnitts in das erste rohrförmige Element, Einbringen des proximalen konischen Abschnitts in das zweite rohrförmige Element und Einbringen des gerade rohrförmigen Abschnitts in das dritte rohrförmige Element in dem entleerten Zustand des Ballons für einen Ballonkatheter, wobei
das Verfahren wenigstens eine der folgenden Bedingungen (1) und (2) erfüllt:
(1) wenigstens ein Abschnitt des Vorsprungabschnitts des distalen konischen Abschnitts steht in dem Einführvorgang in Kontakt mit einer Innenfläche des ersten rohrförmigen Elements; und
(2) wenigstens ein Abschnitt des Vorsprungabschnitts des proximalen konischen Abschnitts steht in dem Einführvorgang in Kontakt mit einer Innenfläche des zweiten rohrförmigen Elements.

## Revendications

1. Ballonnet (2) pour un cathéter à ballonnet (1) comprenant un corps de ballonnet (20) présentant une surface externe et une surface interne, dans lequel le corps de ballonnet présente une partie tubulaire droite (23), une partie conique distale (24) située distalement par rapport à la partie tubulaire droite, et une partie conique proximale (22) située proximalement par rapport à la partie tubulaire droite, la partie conique distale, la partie tubulaire droite et la partie conique proximale présentent une partie en saillie (66) qui fait saillie vers l'extérieur dans une direction radiale (y) à partir de la surface externe du corps de ballonnet et s'étend dans une direction d'axe longitudinal (x) du corps de ballonnet,
la partie en saillie présente une partie pointe (61) dans une section transversale dans la direction radiale du corps de ballonnet,
le corps de ballonnet et la partie en saillie sont moulés d'un seul tenant ;
le ballonnet satisfait à au moins l'un des points (1) et (2) suivants :
(1) dans un état dégonflé du ballonnet pour un cathéter à ballonnet, la partie pointe de la partie en saillie de la partie conique distale est située d'un côté de première direction (C₁) et non d'un côté de seconde direction (C₂), ou située du côté de seconde direction et non du côté de première direction, dans une direction circonférentielle (z) du corps de ballonnet par rapport à une ligne droite L_{d} reliant la partie pointe au niveau d'une extrémité proximale de la partie conique distale à la partie pointe au niveau d'une extrémité distale de la partie conique distale ; et
(2) dans un état dégonflé du ballonnet pour un cathéter à ballonnet, la partie pointe de la partie en saillie de la partie conique proximale est située d'un côté de première direction (C₁) et non d'un côté de seconde direction (C₂), ou située du côté de seconde direction et non du côté de première direction, dans une direction circonférentielle (z) du corps de ballonnet par rapport à une ligne droite Lₚ reliant la partie pointe au niveau d'une extrémité distale de la partie conique proximale à la partie pointe au niveau d'une extrémité proximale de la partie conique proximale ;
**caractérisé en ce que** la partie pointe de la partie en saillie d'au moins l'une de la partie conique distale et de la partie conique proximale est agencée incurvée du côté de première direction ou le côté de seconde direction dans la direction circonférentielle du corps de ballonnet indépendamment par rapport à la ligne droite L_{d} et à la ligne droite Lₚ, respectivement à l'état dégonflé du ballonnet.

2. Ballonnet pour un cathéter à ballonnet selon la revendication 1, dans lequel le ballonnet pour un cathéter à ballonnet est plié à l'état dégonflé du ballonnet pour un cathéter à ballonnet.

3. Ballonnet pour un cathéter à ballonnet selon la revendication 1 ou 2, dans lequel, à l'état dégonflé du ballonnet pour un cathéter à ballonnet, les extrémités de la partie conique distale et de la partie conique proximale du côté de la partie tubulaire droite sont appelées une position de 0 % et les autres extrémités de la partie conique distale et de la partie conique proximale sont appelées une position de 100 % dans la direction d'axe longitudinal du corps de ballonnet, et le ballonnet satisfait à au moins l'un des points (1) et (2) suivants :
(1) la partie pointe de la partie en saillie sur toute l'étendue d'une section d'une position de 20 % à une position de 70 % de la partie conique distale est située du côté de première direction ou du côté de seconde direction par rapport à la ligne droite L_{d}, et la partie pointe de la partie en saillie dans une section d'une position de 90 % à la position de 100 % de la partie conique distale n'est située ni du côté de première direction ni du côté de seconde direction par rapport à la ligne droite L_{d} ; et
(2) la partie pointe de la partie en saillie sur toute l'étendue d'une section d'une position de 20 % à une position de 70 % de la partie conique proximale est située du côté de la première direction ou du côté de la seconde direction par rapport à la ligne droite Lₚ, et la partie pointe de la partie en saillie dans une section d'une position de 90 % à la position de 100 % de la partie conique proximale n'est située ni du côté de première direction ni du côté de seconde direction par rapport à la ligne droite Lₚ.

4. Ballonnet pour un cathéter à ballonnet selon la revendication 3, satisfaisant à au moins l'un des points (1) et (2) suivants :
(1) une distance de la ligne droite L_{d} à la partie pointe de la partie en saillie au niveau d'une position de 40 % de la partie conique distale est plus longue d'au moins 1,2 fois qu'une distance de la ligne droite L_{d} à la partie pointe de la partie en saillie au niveau d'une position de 60 % de la partie conique distale ; et
(2) une distance de la ligne droite Lₚ à la partie pointe de la partie en saillie au niveau d'une position de 40 % de la partie conique proximale est plus longue d'au moins 1,2 fois qu'une distance de la ligne droite Lₚ à la partie pointe de la partie en saillie au niveau d'une position de 60 % de la partie conique proximale.

5. Ballonnet pour un cathéter à ballonnet selon l'une quelconque des revendications 1 à 4, satisfaisant à au moins l'un des points suivants (1) et (2) à l'état dégonflé du ballonnet pour un cathéter à ballonnet :
(1) la partie pointe de la partie en saillie sur toute l'étendue d'une section d'une position de 20 % à une position de 70 % de la partie conique distale est située à l'intérieur ou au niveau de la même position dans la direction radiale du corps de ballonnet par rapport à une surface incurvée imaginaire obtenue en faisant tourner la ligne droite L_{d} autour d'un axe central du corps de ballonnet ; et
(2) la partie pointe de la partie en saillie sur toute l'étendue d'une section d'une position de 20 % à une position de 70 % de la partie conique proximale est située à l'intérieur ou au niveau de la même position dans la direction radiale du corps de ballonnet par rapport à une surface incurvée imaginaire obtenue en faisant tourner la ligne droite Lₚ autour d'un axe central du corps de ballonnet.

6. Ballonnet pour un cathéter à ballonnet selon l'une quelconque des revendications 1 à 5, dans lequel la partie pointe de la partie en saillie de la partie conique distale, la partie pointe de la partie en saillie de la partie tubulaire droite et la partie pointe de la partie en saillie de la partie conique proximale sont situées au niveau de la même position dans la direction circonférentielle du corps de ballonnet dans un état gonflé du ballonnet pour un cathéter à ballonnet.

7. Ballonnet pour un cathéter à ballonnet selon l'une quelconque des revendications 1 à 6, dans lequel le corps de ballonnet présente une partie formant une aile (28) qui forme une aile (29) à l'état dégonflé, et la partie en saillie est située à l'extérieur de la partie formant une aile.

8. Ballonnet pour un cathéter à ballonnet selon l'une quelconque des revendications 1 à 7, dans lequel la partie en saillie de la partie conique distale, la partie en saillie de la partie tubulaire droite et la partie en saillie de la partie conique proximale s'étendent en continu dans la direction d'axe longitudinal du corps de ballonnet.

9. Ballonnet pour un cathéter à ballonnet selon l'une quelconque des revendications 1 à 8, dans lequel la partie en saillie est composée du même matériau que le corps de ballonnet.

10. Procédé de fabrication du ballonnet pour un cathéter à ballonnet selon l'une quelconque des revendications 1 à 9, comprenant :
la préparation d'un premier organe tubulaire (310), d'un deuxième organe tubulaire (320), et d'un troisième organe tubulaire (330), chacun présentant un espace interne s'étendant dans une direction d'axe longitudinal (x) ;
la préparation d'un ballonnet (2) pour un cathéter à ballonnet (1) présentant un corps de ballonnet (20) présentant une surface externe et une surface interne, le corps de ballonnet présentant une partie tubulaire droite (23), une partie conique distale (24) située distalement à la partie tubulaire droite, et une partie conique proximale (22) située proximalement à la partie tubulaire droite, et la partie conique distale, la partie tubulaire droite, et la partie conique proximale présentant une partie en saillie (60) qui fait saillie vers l'extérieur dans une direction radiale à partir de la surface externe du corps de ballonnet et s'étend dans une direction d'axe longitudinal du corps de ballonnet ; et
le placement de la partie conique distale dans le premier organe tubulaire, le placement de la partie conique proximale dans le deuxième organe tubulaire, et le placement de la partie tubulaire droite dans le troisième organe tubulaire à l'état dégonflé du ballonnet pour un cathéter à ballonnet, dans lequel
le procédé satisfait à au moins l'un des points (1) et (2) suivants :
(1) au moins une partie de la partie en saillie de la partie conique distale est en contact avec une surface interne du premier organe tubulaire lors du processus de placement ; et
(2) au moins une partie de la partie en saillie de la partie conique proximale est en contact avec une surface interne du deuxième organe tubulaire lors du processus de placement.
